# EUROPÄISCHE PATENTSCHRIFT

(11) **EP 2 922 932 B1**
(45) Veröffentlichungstag und Bekanntmachung des Hinweises auf die Patenterteilung: **12.06.2019**
(21) Anmeldenummer: 13782978.4
(22) Anmeldetag: 25.10.2013
(51) Int. Cl.: C09K 11/06, H01L 51/50, H05B 33/22

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 23.11.2012 EP 12007922
(43) Veröffentlichungstag der Anmeldung: 30.09.2015
(73) Patentinhaber: Merck Patent GmbH, 64293 Darmstadt (DE)
(72) Erfinder: BUESING, Arne, 65929 Frankfurt am Main (DE); MARTYNOVA, Irina, 64347 Griesheim (DE); MUJICA-FERNAUD, Teresa, 64283 Darmstadt (DE); VOGES, Frank, 67098 Bad Duerkheim (DE)
(86) Internationale Anmeldenummer: PCT/EP2013/003218
(87) Internationale Veröffentlichungsnummer: WO 2014/079527

(56) Entgegenhaltungen:
- EP-A1- 2 399 906
- WO-A1-2008/127057
- WO-A1-2013/129835
- JP-A- 2007 110 093
- JP-A- 2012 062 450
- US-A1- 2009 284 143
- US-A1- 2011 248 247

## Beschreibung

Die vorliegende Anmeldung betrifft eine Verbindung der Formel (I), welche eine Carbazoleinheit und eine Arylaminoeinheit enthält. Die Verbindung eignet sich zur Verwendung als funktionelles Material in einer elektronischen Vorrichtung, bevorzugt als funktionelles Material mit lochtransportierenden Eigenschaften. Weiterhin betrifft die vorliegende Anmeldung ein Verfahren zur Herstellung der Verbindung der Formel (I).

Unter elektronischen Vorrichtungen im Sinne dieser Anmeldung werden insbesondere sogenannte organische elektronische Vorrichtungen verstanden (organic electronic devices), welche organische Halbleitermaterialien als Funktionsmaterialien enthalten. Nochmals insbesondere werden darunter organische Elektrolumineszenzvorrichtungen (OLEDs) und andere elektronische Vorrichtungen verstanden, welche im Folgenden bei der detaillierten Beschreibung der Erfindung aufgeführt sind.

Der genaue Aufbau von OLEDs ist unter anderem in US 4539507, US 5151629, EP 0676461 und WO 98/27136 beschrieben. Allgemein werden unter der Bezeichnung OLED elektronische Vorrichtungen verstanden, welche mindestens ein organisches Material enthalten und unter Anlegen einer elektrischen Spannung Licht emittieren.

Bei elektronischen Vorrichtungen, insbesondere OLEDs, besteht großes Interesse an der Verbesserung der Leistungsdaten, insbesondere Lebensdauer und Effizienz und Betriebsspannung. Eine wichtige Rolle spielen dabei Schichten mit lochtransportierender Funktion in der elektronischen Vorrichtung. Weiterhin besteht großes Interesse an der Bereitstellung von Verbindungen mit vorteilhaften Glasbildungseigenschaften, insbesondere hoher Glasübergangstemperatur.

Zur Lösung dieser technischen Aufgabe werden kontinuierlich neue Materialien mit lochtransportierenden Eigenschaften gesucht.

Im Stand der Technik bekannt ist die Verwendung von Triarylamin-Verbindungen in elektronischen Vorrichtungen. Diese können Mono-Triarylamine, wie beispielsweise in JP 1995/053955, WO 2006/123667 und JP 2010/222268 beschrieben, oder Bis- oder höherwertige Amine darstellen, wie beispielsweise in US 7504163 oder US 2005/0184657 beschrieben. Bekannte Beispiele sind unter anderem Tris-p-biphenyl-amin, N,N'-Di-1-Naphthyl-N,N'-diphenyl-1,1'-biphenyl-4,4'-diamin (NPB) und 4,4',4"-Tris-(3-methylphenylphenylamino)triphenylamin (MTDATA).

Ebenfalls bekannt ist die Verwendung von Verbindungen, die eine Carbazoleinheit aufweisen. Solche Verbindungen werden insbesondere in emittierenden Schichten als Hostmaterialien eingesetzt. Bekannt ist unter anderem die Verbindung CBP (N,N-Biscarbazolylbiphenyl) oder Verbindungen gemäß WO 2005/039246, US 2005/0069729, JP 2004/288381, EP 1205527 oder WO 2008/086851.

Weiterhin ist im Stand der Technik die Verwendung von lochtransportierenden Verbindungen bekannt, die sowohl Carbazolgruppen als auch Triarylaminogruppen aufweisen, und bei denen die Gruppen über Linker-Einheiten wie Phenylen verbunden sind (JP 2007-110093 und US 2011/248247 A1). In den Ausführungsbeispielen der JP 2007-110093 sind grundsätzlich drei, d.h. mehrere Carbazolgruppen pro Molekül vorhanden. Die Verbindungen eignen sich als Funktionsmaterialien zur Verwendung in OLEDs.

Es besteht jedoch weiterhin Verbesserungsbedarf bezüglich der im Stand der Technik bekannten Verbindungen, insbesondere für die Verwendung der Verbindungen in Lochtransport- und Elektronenblockierschichten, und nochmals insbesondere in den Punkten Effizienz und Lebensdauer von Vorrichtungen enthaltend die Verbindungen.

Überraschend wurde nun gefunden, dass durch die Verwendung einer Linkereinheit bestehend aus drei oder mehr Phenyleneinheiten zwischen einer einzigen Triarylaminogruppe und einer einzigen Carbazolgruppe Verbindungen mit hervorragenden Eigenschaften erhalten werden. Bei Verwendung in elektronischen Vorrichtungen werden mit solchen Verbindungen hervorragende Lebensdauern und Leistungseffizienzen der Vorrichtungen erzielt.

Gegenstand der vorliegenden Anmeldung ist somit eine Verbindung einer Formel (I), definiert wie in Anspruch 1:

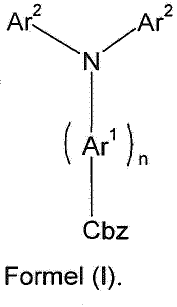

Unter einer Carbazolgruppe werden im Sinne der vorliegenden Anmeldung auch Carbazolgruppen verstanden, bei denen ein oder mehrere Kohlenstoffatome der aromatischen Sechsringe durch Stickstoff ausgetauscht sind. Weiterhin werden darunter auch Carbazolgruppen verstanden, bei denen der Carbazol-Fünfring zu einem Sechsring erweitert ist, so dass gegenüber vom Stickstoffatom beispielsweise eine Methylen-, Silylen-, Sauerstoff- oder Schwefelbrücke angeordnet ist. Dadurch entsteht im ersten Fall beispielsweise eine Einheit, die auch als Dihydroacridin bezeichnet wird. Weiterhin werden darunter auch Carbazolgruppen mit ankondensierten Gruppen, wie beispielsweise Indenocarbazole oder Indolocarbazole, verstanden.

Unter dem Carbazol-Stickstoffatom wird im Sinne der vorliegenden Anmeldung das Stickstoffatom des Fünfrings bzw. des erweiterten Fünfrings des Carbazols verstanden.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

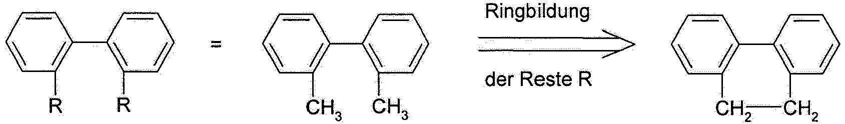

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

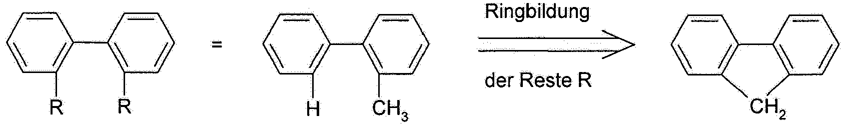

Weiterhin ist bevorzugt, dass die Verbindung der Formel (I) neben der gezeigten Arylaminogruppe keine weitere Arylaminogruppe enthält. Unter einer Arylaminogruppe wird im Sinne dieser Anmeldung eine Gruppe verstanden, in der mindestens eine Arylgruppe oder Heteroarylgruppe an ein dreibindiges Stickstoffatom gebunden ist. Wie die Gruppe weiter aufgebaut ist, oder welche weiteren Gruppen sie umfasst, ist für die Definition unerheblich. Besonders bevorzugt enthält die Verbindung der Formel (I) neben der gezeigten Arylaminogruppe keine weitere Aminogruppe.

Weiterhin ist es bevorzugt, dass die Verbindung der Formel (I) keine kondensierte Aryl- oder Heteroarylgruppe mit mehr als 14 aromatischen Ringatomen enthält. Besonders bevorzugt enthält sie keine kondensierte Aryl- oder Heteroarylgruppe mit mehr als 10 aromatischen Ringatomen.

Weiterhin ist R¹ bevorzugt bei jedem Auftreten gleich oder verschieden H, D, F, CN, Si(R³)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R³ substituiert sein können und wobei in den oben genannten Gruppen eine oder mehrere CH₂-Gruppen durch -C=C-, - R³C=CR³-, Si(R³)₂, C=O, C=NR³, -NR³-, -O-, -S-, -C(=O)O- oder - C(=O)NR³- ersetzt sein können, oder ein aromatisches oder heteroaromatisches Ringsystem mit 5 bis 20 aromatischen Ringatomen, das jeweils mit einem oder mehreren Resten R³ substituiert sein kann, wobei zwei oder mehr Reste R¹ miteinander verknüpft sein können und einen Ring bilden können.

Weiterhin ist R² bevorzugt bei jedem Auftreten gleich oder verschieden H, D, F, CN, Si(R³)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R³ substituiert sein können und wobei in den oben genannten Gruppen eine oder mehrere CH₂-Gruppen durch -C=C-, - R³C=CR³-, Si(R³)₂, C=O, C=NR³, -NR³-, -O-, -S-, -C(=O)O- oder - C(=O)NR³- ersetzt sein können, oder ein aromatisches oder heteroaromatisches Ringsystem mit 5 bis 20 aromatischen Ringatomen, das jeweils mit einem oder mehreren Resten R³ substituiert sein kann, wobei zwei oder mehr Reste R² miteinander verknüpft sein können und einen Ring bilden können.

Weiterhin ist R³ bevorzugt bei jedem Auftreten gleich oder verschieden H, D, F, CN, Si(R⁴)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R⁴ substituiert sein können und wobei in den oben genannten Gruppen eine oder mehrere CH₂-Gruppen durch -C=C-, - R⁴C=CR⁴-, Si(R⁴)₂, C=O, C=NR⁴, -NR⁴-, -O-, -S-, -C(=O)O- oder - C(=O)NR⁴- ersetzt sein können, oder ein aromatisches oder heteroaromatisches Ringsystem mit 5 bis 20 aromatischen Ringatomen, das jeweils mit einem oder mehreren Resten R⁴ substituiert sein kann, wobei zwei oder mehr Reste R³ miteinander verknüpft sein können und einen Ring bilden können.

Es gilt bevorzugt, dass Cbz gewählt ist aus Gruppen der Formel (Cbz) 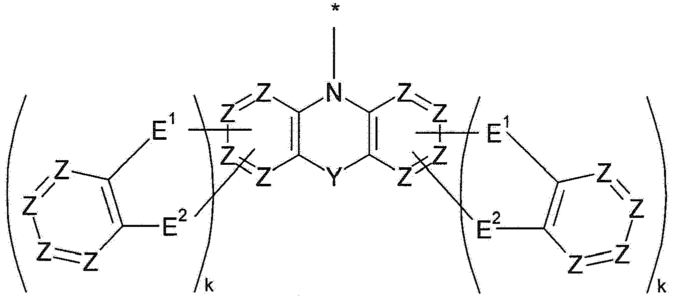 Formel (Cbz), wobei gilt:
- Z: ist gleich CR¹, wobei Z gleich C ist, wenn eine Gruppe E¹ oder E² gebunden ist;
- E¹, E²: ist bei jedem Auftreten gleich oder verschieden gewählt aus einer Einfachbindung, C(R¹)₂, Si(R¹)₂, C=O, O, S, S=O, SO₂ und NR¹, wobei E¹ und E² nicht beide eine Einfachbindung sein können;
- Y: ist eine Einfachbindung;
- k: ist bei jedem Auftreten gleich oder verschieden 0 oder 1;
wobei die Gruppe der Formel (Cbz) über die mit * markierte Bindung gebunden ist.

Dabei gilt bevorzugt, dass die Summe der Indices k in der Gruppe der Formel (Cbz) gleich 0 oder 1 ist. Besonders bevorzugt ist sie gleich 0, d.h. alle Indices k sind gleich 0.

Für die Gruppen E¹ und E² in der Gruppe der Formel (Cbz) gilt, dass sie bevorzugt an benachbarte Gruppen Z am Sechsring binden.

Besonders bevorzugt binden die Gruppen E¹ und E² in der Gruppe der Formel (Cbz) an die Positionen, die sich in para-Stellung zu den Gruppen Y und N an den aromatischen Sechsringen befinden. Diese sind im folgenden Schema mit dem Zeichen # markiert:

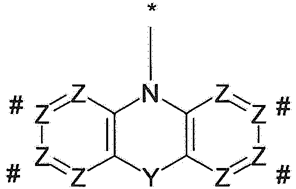

Für die Gruppen E¹ und E² in der Gruppe der Formel (Cbz) gilt, dass innerhalb einer Einheit in Klammern mit Index k bevorzugt eine von E¹ und E² eine Einfachbindung ist, und die andere gewählt ist aus C(R¹)₂, Si(R¹)₂, C=O, O, S, S=O, SO₂ und NR¹, bevorzugt aus C(R¹)₂, O und S, und besonders bevorzugt C(R¹)₂ ist.

Bevorzugt ist weiterhin, bevorzugt in Kombination mit den oben angeführten Bevorzugungen, dass Cbz gewählt ist aus Gruppen der Formel (Cbz-1) bis (Cbz-10) 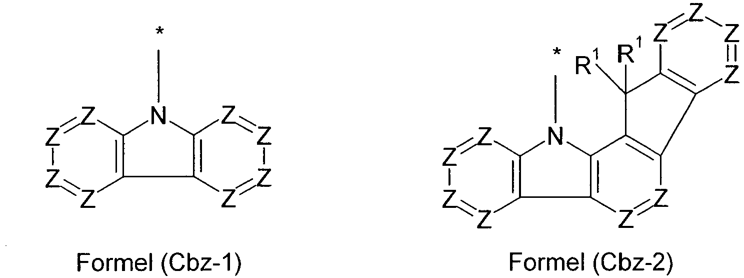 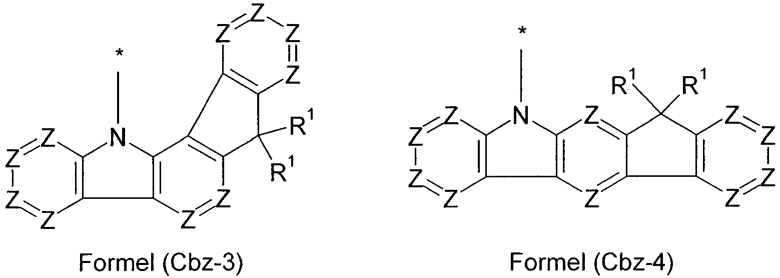 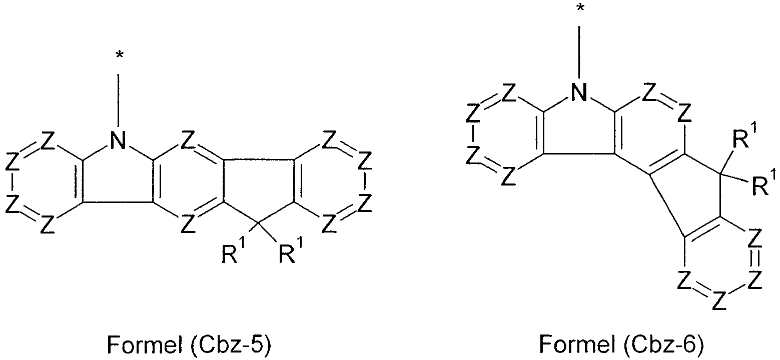 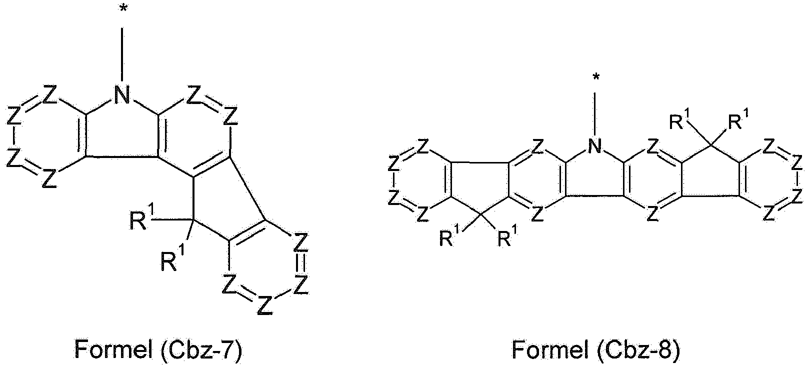 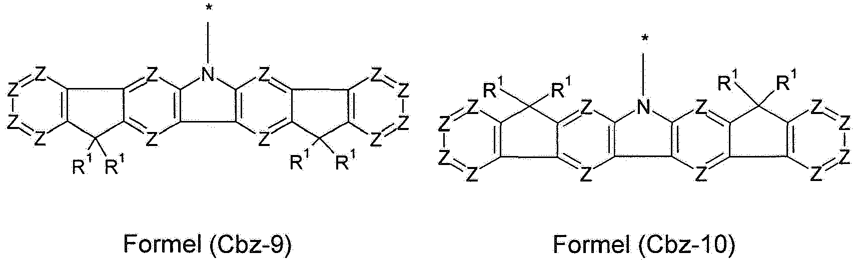 wobei die auftretenden Symbole wie oben definiert sind und die Gruppe Cbz über die mit * markierte Bindung gebunden ist.

Weiterhin gelten bevorzugt für die Gruppen (Cbz-1) bis (Cbz-10) die oben angegebenen bevorzugten Ausführungsformen von Gruppen.

Weiterhin ist es für Gruppen (Cbz-1) bis (Cbz-10) bevorzugt, dass R¹, R² und R³ so definiert sind wie oben als bevorzugt angegeben.

Unter den oben angegebenen Formeln sind die Formeln (Cbz-1) und (Cbz-4) besonders bevorzugt.

Bevorzugt ist Ar¹ gewählt aus ortho-Phenylen, meta-Phenylen und para-Phenylen, die jeweils mit einem oder mehreren Resten R² substituiert sein können, und wobei einzelne Gruppen Ar¹ miteinander auch über Reste R² verbunden sein können.

Es ist besonders bevorzugt für die Ausführungsform, in der Gruppen Ar¹ miteinander über Reste R² verbunden sind, dass dabei aus zwei mit Resten R² substituierten Phenylgruppen Fluorengruppen, Dibenzofurangruppen oder Dibenzothiophengruppen gebildet werden, bevorzugt Fluorengruppen.

Weiterhin ist es bevorzugt, dass mindestens eine Gruppe Ar¹ gewählt ist aus meta-Phenylen, wobei die Gruppen mit einem oder mehreren Resten R² substituiert sein können. Besonders bevorzugt sind mindestens zwei Gruppen Ar¹ gewählt aus ortho-Phenylen oder meta-Phenylen, bevorzugt aus meta-Phenylen, wobei die Gruppen mit einem oder mehreren Resten R² substituiert sein können.

Weiterhin ist es bevorzugt, dass n gleich 3 ist, und die mittlere der drei Gruppen Ar¹ gleich meta-Phenylen oder ortho-Phenylen ist, das mit einem oder mehreren Resten R² substituiert sein kann.

Weiterhin bevorzugt ist Ar² bei jedem Auftreten gleich oder verschieden gewählt aus einem aromatischen oder heteroaromatischen Ringsystem mit 6 bis 24 aromatischen Ringatomen, das mit einem oder mehreren Resten R² substituiert sein kann. Ganz besonders bevorzugt sind darunter Phenyl, Biphenyl, Naphthyl, Terphenyl, Fluorenyl, Spirobifluoren, Indenofluorenyl, Dibenzofuran, und Dibenzothiophen, die mit einem oder mehreren Resten R² substituiert sein können.

Bevorzugte Verbindungen der Formel (I) entsprechen der folgenden Formel (I-A)

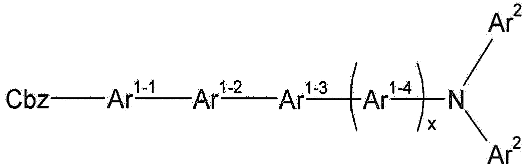

Formel (I-A), wobei gilt:
Cbz entspricht Formel (Cbz-1) oder (Cbz-4), wie oben definiert;
- Ar¹⁻¹ bis Ar¹⁻⁴: sind definiert wie Ar¹ oben;
- Ar²: ist definiert wie Ar² oben;
- x: ist 0.

Für den Fall von x=0 ist die Gruppe Ar¹⁻⁴ nicht vorhanden (n. v.), und Ar¹⁻³ und N sind direkt miteinander verbunden.

Es gelten für Formel (I-A) die oben angegebenen bevorzugten Ausführungsformen von Gruppen ebenfalls als bevorzugt.

Bevorzugt ist Ar¹ gewählt aus ortho-Phenylen, meta-Phenylen und para-Phenylen, die jeweils mit einem oder mehreren Resten R² substituiert sein können, und wobei einzelne Gruppen Ar¹ miteinander auch über Reste R² verbunden sein können.

Besonders bevorzugte Ausführungsformen von Verbindungen der Formel (I-A) entsprechen den folgenden Formeln. Formeln (I-A-53) bis (I-A-212) sind dabei nicht anspruchsgemäß.

| Formel (I-A- | Cbz | Ar¹⁻¹ | Ar¹⁻² | Ar¹⁻³ | Ar¹⁻⁴ |
|---|---|---|---|---|---|
| 1) | (Cbz-1) | p- | p- | m- | n.v. |
| 2) | " | p- | p- | o- | n.v. |
| 3) | " | p- | m- | p- | n.v. |
| 4) | " | p- | m- | m- | n.v. |
| 5 | " | p- | m- | o- | n.v. |
| 6) | " | p- | o- | p- | n.v. |
| 7) | " | p- | o- | m- | n.v. |
| 8) | " | p- | o- | o- | n.v. |
| 9) | " | m- | p- | p- | n.v. |
| 10) | " | m- | p- | m- | n.v. |
| 11) | " | m- | p- | o- | n.v. |
| 12) | " | m- | m- | p- | n.v. |
| 13 | " | m- | m- | m- | n.v. |
| 14) | " | m- | m- | o- | n.v. |
| 15) | " | m- | o- | p- | n.v. |
| 16) | " | m- | o- | m- | n.v. |
| 17) | " | m- | o- | o- | n.v. |
| 18) | " | o- | p- | p- | n.v. |
| 19) | " | o- | p- | m- | n.v. |
| 20) | " | o- | p- | o- | n.v. |
| 21) | " | o- | m- | p- | n.v. |
| 22) | " | o- | m- | m- | n.v. |
| 23) | " | o- | m- | o- | n.v. |
| 24) | " | o- | o- | p- | n.v. |
| 25) | " | o- | o- | m- | n.v. |
| 26) | " | o- | o- | o- | n.v. |
| 27) | (Cbz-4) | p- | p- | m- | n.v. |
| 28) | " | p- | p- | o- | n.v. |
| 29) | " | p- | m- | p- | n.v. |
| 30) | " | p- | m- | m- | n.v. |
| 31) | " | p- | m- | o- | n.v. |
| 32) | " | p- | o- | p- | n.v. |
| 33) | " | p- | o- | m- | n.v. |
| 34) | " | p- | o- | o- | n.v. |
| 35 | " | m- | p- | p- | n.v. |
| 36) | " | m- | p- | m- | n.v. |
| 37) | " | m- | p- | o- | n.v. |
| 38) | " | m- | m- | p- | n.v. |
| 39) | " | m- | m- | m- | n.v. |
| 40) | " | m- | m- | o- | n.v. |
| 41) | " | m- | o- | p- | n.v. |
| 42) | " | m- | o- | m- | n.v. |
| 43 | " | m- | o- | o- | n.v. |
| 44) | " | o- | p- | p- | n.v. |
| 45) | " | o- | p- | m- | n.v. |
| 46) | " | o- | p- | o- | n.v. |
| 47) | " | o- | m- | p- | n.v. |
| 48) | " | o- | m- | m- | n.v. |
| 49) | " | o- | m- | o- | n.v. |
| 50) | " | o- | o- | p- | n.v. |
| 51) | " | o- | o- | m- | n.v. |
| 52) | " | o- | o- | o- | n.v. |
| 53) | (Cbz-1) | p- | p- | m- | p- |
| 54) | " | p- | p- | o- | p- |
| 55) | " | p- | m- | p- | p- |
| 56) | " | p- | m- | m- | p- |
| 57) | " | p- | m- | o- | p- |
| 58) | " | p- | o- | p- | p- |
| 59) | " | p- | o- | m- | p- |
| 60) | " | p- | o- | o- | p- |
| 61) | " | m- | p- | p- | p- |
| 62) | " | m- | p- | m- | p- |
| 63) | " | m- | p- | o- | p- |
| 64) | " | m- | m- | p- | p- |
| 65) | " | m- | m- | m- | p- |
| 66) | " | m- | m- | o- | p- |
| 67) | " | m- | o- | p- | p- |
| 68) | " | m- | o- | m- | p- |
| 69) | " | m- | o- | o- | p- |
| 70) | " | o- | p- | p- | p- |
| 71) | " | o- | p- | m- | p- |
| 72) | " | o- | p- | o- | p- |
| 73) | " | o- | m- | p- | p- |
| 74) | " | o- | m- | m- | p- |
| 75) | " | o- | m- | o- | p- |
| 76) | " | o- | o- | p- | p- |
| 77) | " | o- | o- | m- | p- |
| 78) | " | o- | o- | o- | p- |
| 79) | " | p- | p- | p- | m- |
| 80) | " | p- | p- | m- | m- |
| 81) | " | p- | p- | o- | m- |
| 82) | " | p- | m- | p- | m- |
| 83) | " | p- | m- | m- | m- |
| 84 | " | p- | m- | o- | m- |
| 85) | " | p- | o- | p- | m- |
| 86) | " | p- | o- | m- | m- |
| 87) | " | p- | o- | o- | m- |
| 88) | " | m- | p- | p- | m- |
| 89) | " | m- | p- | m- | m- |
| 90) | " | m- | p- | o- | m- |
| 91) | " | m- | m- | p- | m- |
| 92) | " | m- | m- | m- | m- |
| 93) | " | m- | m- | o- | m- |
| 94) | " | m- | o- | p- | m- |
| 95) | " | m- | o- | m- | m- |
| 96) | " | m- | o- | o- | m- |
| 97) | " | o- | p- | p- | m- |
| 98) | " | o- | p- | m- | m- |
| 99) | " | o- | p- | o- | m- |
| 100) | " | o- | m- | p- | m- |
| 101) | " | o- | m- | m- | m- |
| 102) | " | o- | m- | o- | m- |
| 103) | " | o- | o- | p- | m- |
| 104) | " | o- | o- | m- | m- |
| 105) | " | o- | o- | o- | m- |
| 106) | " | p- | p- | p- | o- |
| 107) | " | p- | p- | m- | o- |
| 108) | " | p- | p- | o- | o- |
| 109 | " | p- | m- | p- | o- |
| 110) | " | p- | m- | m- | o- |
| 111) | " | p- | m- | o- | o- |
| 112) | " | p- | o- | p- | o- |
| 113) | " | p- | o- | m- | o- |
| 114) | " | p- | o- | o- | o- |
| 115) | " | m- | p- | p- | o- |
| 116) | " | m- | p- | m- | o- |
| 117) | " | m- | p- | o- | o- |
| 118) | " | m- | m- | p- | o- |
| 119) | " | m- | m- | m- | o- |
| 120) | " | m- | m- | o- | o- |
| 121) | " | m- | o- | p- | o- |
| 122) | " | m- | o- | m- | o- |
| 123) | " | m- | o- | o- | o- |
| 124) | " | o- | p- | p- | o- |
| 125) | " | o- | p- | m- | o- |
| 126) | " | o- | p- | o- | o- |
| 127) | " | o- | m- | p- | o- |
| 128) | " | o- | m- | m- | o- |
| 129) | " | o- | m- | o- | o- |
| 130) | " | o- | o- | p- | o- |
| 131) | " | o- | o- | m- | o- |
| 132) | " | o- | o- | o- | o- |
| 133) | (Cbz-4) | p- | p- | m- | p- |
| 134) | " | p- | p- | o- | p- |
| 135) | " | p- | m- | p- | p- |
| 136) | " | p- | m- | m- | p- |
| 137) | " | p- | m- | o- | p- |
| 138) | " | p- | o- | p- | p- |
| 139) | " | p- | o- | m- | p- |
| 140) | " | p- | o- | o- | p- |
| 141) | " | m- | p- | p- | p- |
| 142) | " | m- | p- | m- | p- |
| 143) | " | m- | p- | o- | p- |
| 144) | " | m- | m- | p- | p- |
| 145) | " | m- | m- | m- | p- |
| 146) | " | m- | m- | o- | p- |
| 147) | " | m- | o- | p- | p- |
| 148) | " | m- | o- | m- | p- |
| 149) | " | m- | o- | o- | p- |
| 150) | " | o- | p- | p- | p- |
| 151) | " | o- | p- | m- | p- |
| 152) | " | o- | p- | o- | p- |
| 153) | " | o- | m- | p- | p- |
| 154) | " | o- | m- | m- | p- |
| 155) | " | o- | m- | o- | p- |
| 156) | " | o- | o- | p- | p- |
| 157) | " | o- | o- | m- | p- |
| 158) | " | o- | o- | o- | p- |
| 159) | " | p- | p- | p- | m- |
| 160) | " | p- | p- | m- | m- |
| 161) | " | p- | p- | o- | m- |
| 162) | " | p- | m- | p- | m- |
| 163) | " | p- | m- | m- | m- |
| 164) | " | p- | m- | o- | m- |
| 165) | " | p- | o- | p- | m- |
| 166) | " | p- | o- | m- | m- |
| 167) | " | p- | o- | o- | m- |
| 168) | " | m- | p- | p- | m- |
| 169) | " | m- | p- | m- | m- |
| 170) | " | m- | p- | o- | m- |
| 171) | " | m- | m- | p- | m- |
| 172) | " | m- | m- | m- | m- |
| 173) | " | m- | m- | o- | m- |
| 174) | " | m- | o- | p- | m- |
| 175) | " | m- | o- | m- | m- |
| 176) | " | m- | o- | o- | m- |
| 177) | " | o- | p- | p- | m- |
| 178) | " | o- | p- | m- | m- |
| 179) | " | o- | p- | o- | m- |
| 180) | " | o- | m- | p- | m- |
| 181) | " | o- | m- | m- | m- |
| 182) | " | o- | m- | o- | m- |
| 183) | " | o- | o- | p- | m- |
| 184) | " | o- | o- | m- | m- |
| 185) | " | o- | o- | o- | m- |
| 186) | " | p- | p- | p- | o- |
| 187) | " | p- | p- | m- | o- |
| 188) | " | p- | p- | o- | o- |
| 189) | " | p- | m- | p- | o- |
| 190) | " | p- | m- | m- | o- |
| 191) | " | p- | m- | o- | o- |
| 192) | " | p- | o- | p- | o- |
| 193) | " | p- | o- | m- | o- |
| 194) | " | p- | o- | o- | o- |
| 195) | " | m- | p- | p- | o- |
| 196) | " | m- | p- | m- | o- |
| 197) | " | m- | p- | o- | o- |
| 198) | " | m- | m- | p- | o- |
| 199) | " | m- | m- | m- | o- |
| 200) | " | m- | m- | o- | o- |
| 201) | " | m- | o- | p- | o- |
| 202) | " | m- | o- | m- | o- |
| 203) | " | m- | o- | o- | o- |
| 204) | " | o- | p- | p- | o- |
| 205) | " | o- | p- | m- | o- |
| 206) | " | o- | p- | o- | o- |
| 207) | " | o- | m- | p- | o- |
| 208) | " | o- | m- | m- | o- |
| 209) | " | o- | m- | o- | o- |
| 210) | " | o- | o- | p- | o- |
| 211) | " | o- | o- | m- | o- |
| 212) | " | o- | o- | o- | o- |

In der Tabelle steht "p-" jeweils für para-Phenylen, das mit einem oder mehreren Resten R², wie oben definiert, substituiert sein kann. Entsprechend steht "m-" für meta-Phenylen, das mit einem oder mehreren Resten R², wie oben definiert, substituiert sein kann. Entsprechend steht "o-" für ortho-Phenylen, das mit einem oder mehreren Resten R², wie oben definiert, substituiert sein kann.

Ar¹⁻¹ = ortho-Phenylen bezeichnet dabei, dass die Gruppe Ar¹⁻¹ über orthostehende Bindungen mit den benachbarten Gruppen, hier Cbz und Ar¹⁻², verbunden ist. Entsprechendes gilt für meta-Phenylen (meta-stehende Bindungen) und para-Phenylen (para-stehende Bindungen).

Beispiele für Verbindungen gemäß Formel (I) sind in der folgenden Tabelle aufgeführt. Nicht anspruchsgemäße Verbindungen sind mit # markiert.

| | | |
|---|---|---|
| 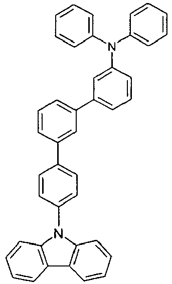 | 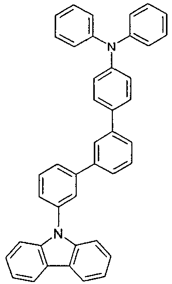 | 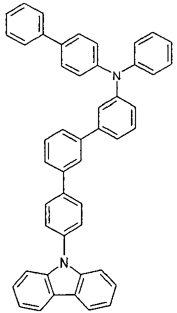 |
| 1 | 2 | 3 |
| 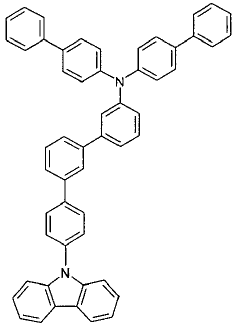 | 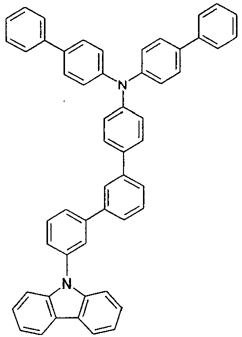 | 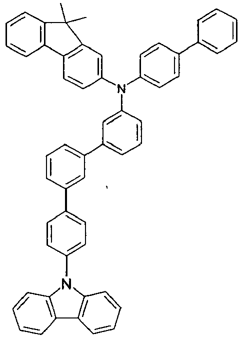 |
| 4 | 5 | 6 |
| 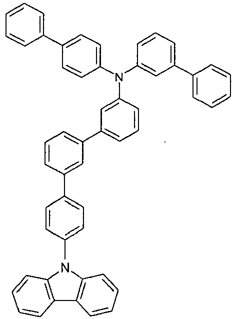 | 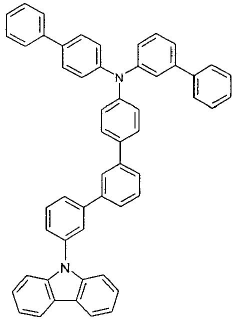 | 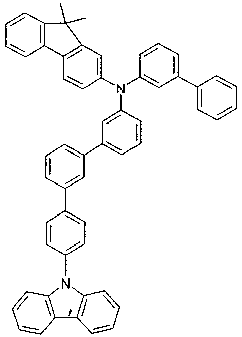 |
| 7 | 8 | 9 |
| 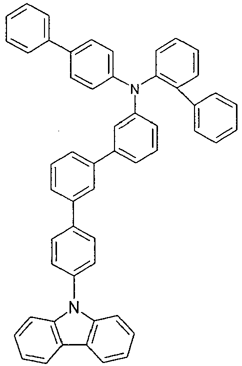 | 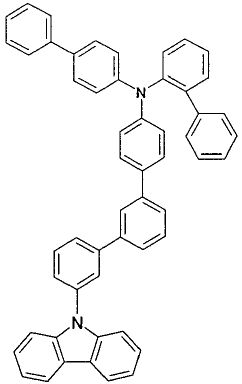 | 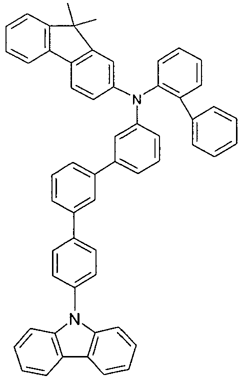 |
| 10 | 11 | 12 |
| 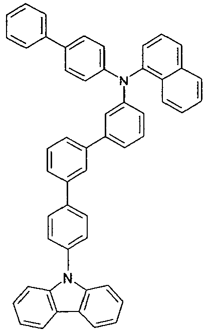 | 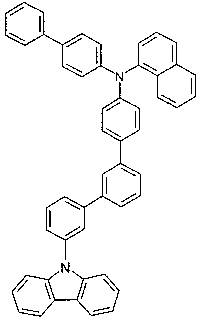 | 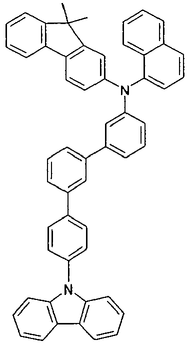 |
| 13 | 14 | 15 |
| 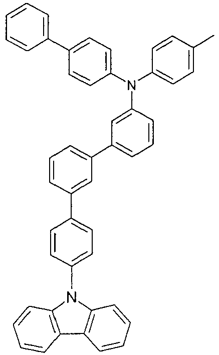 | 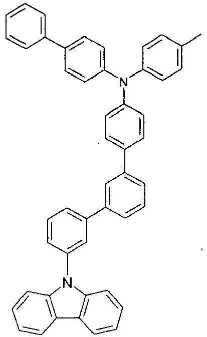 | 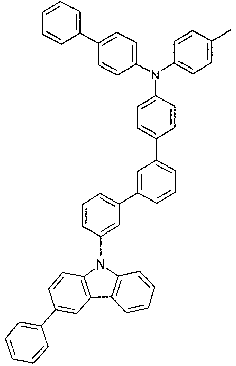 |
| 16 | 17 | 18 |
| 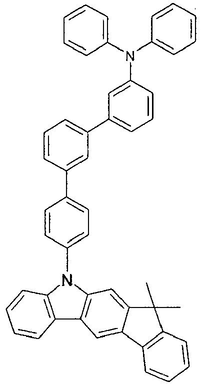 | 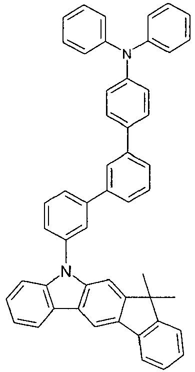 | 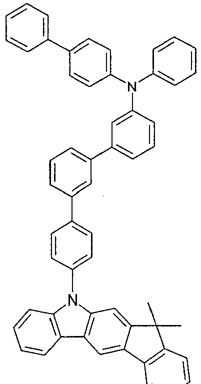 |
| 19 | 20 | 21 |
| 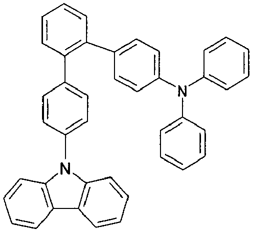 | 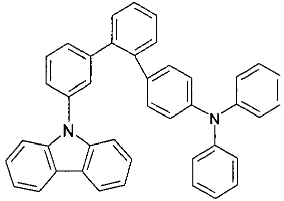 | 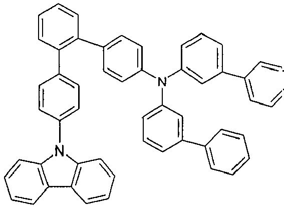 |
| 22 | 23 | 24 |
| 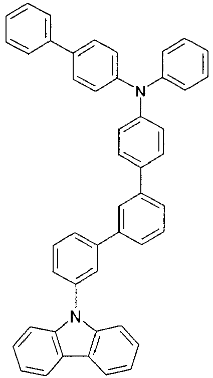 | 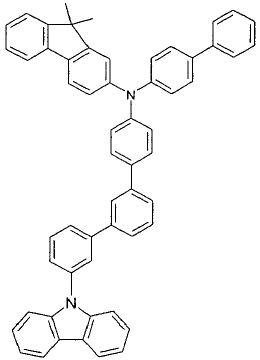 | 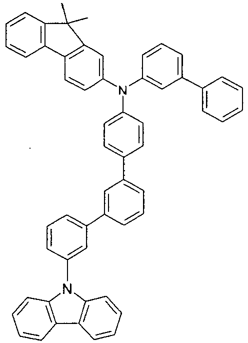 |
| 25 | 26 | 27 |
| 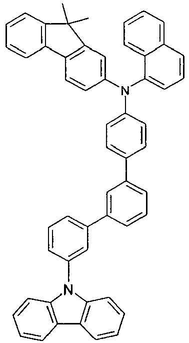 | 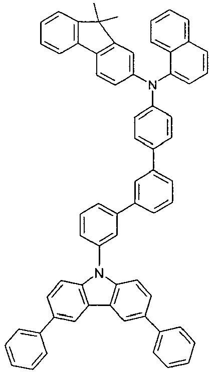 | 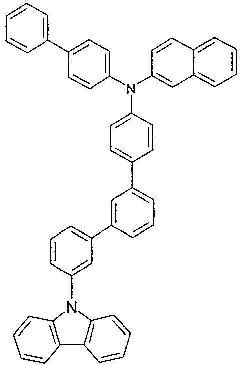 |
| 28 | 29 | 30 |
| 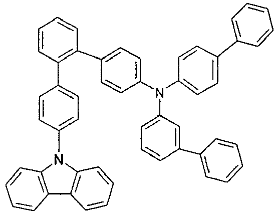 | 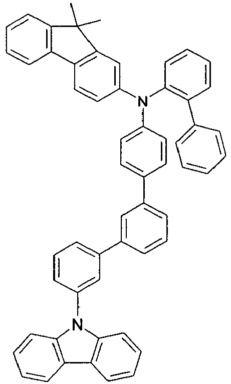 | 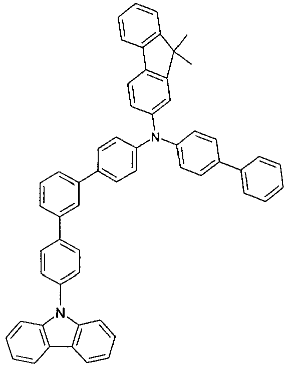 |
| 31 | 32 | 33 |
| 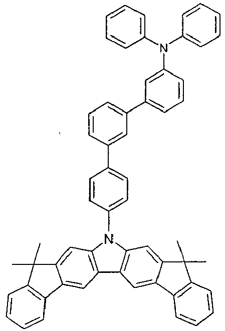 | 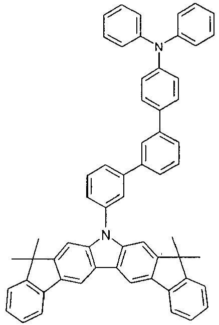 | 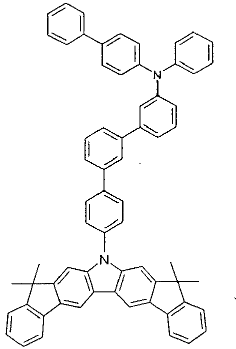 |
| 34 | 35 | 36 |
| 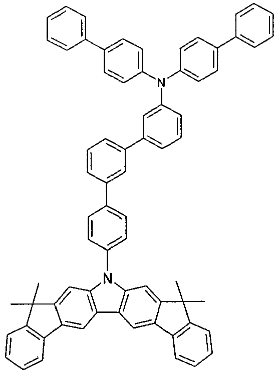 | 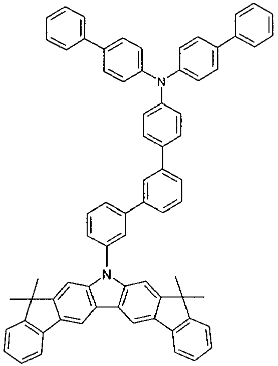 | 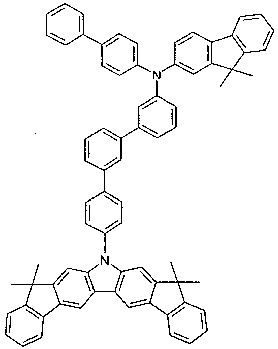 |
| 37 | 38 | 39 |
| 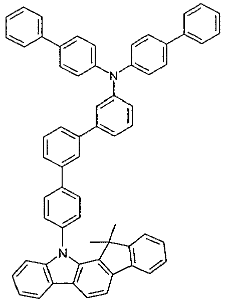 | 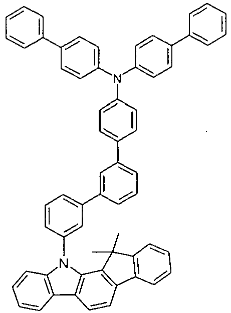 | 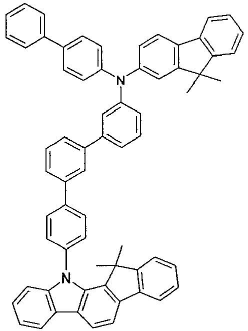 |
| 40 | 41 | 42 |
| 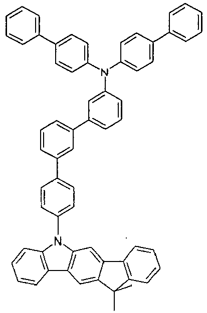 | 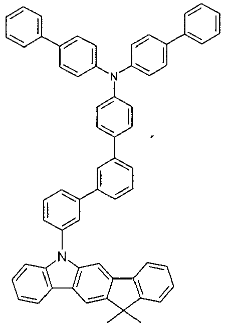 | 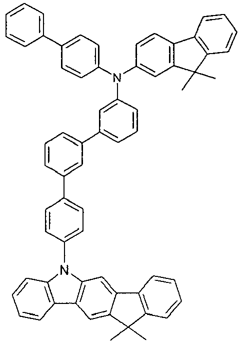 |
| 43 | 44 | 45 |
| 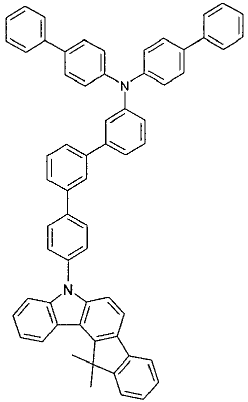 | 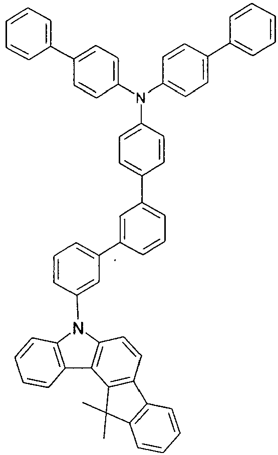 | 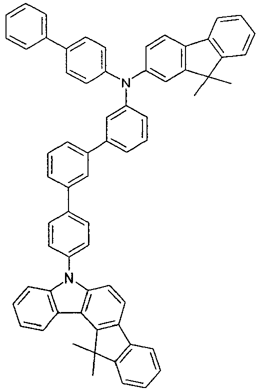 |
| 46 | 47 | 48 |
| 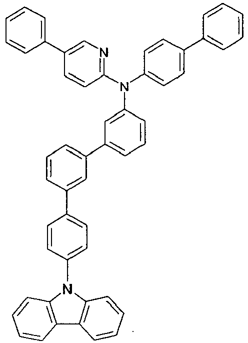 | 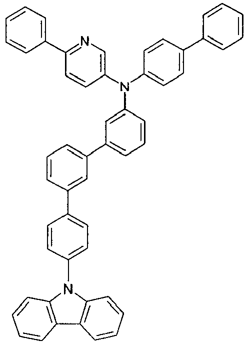 | 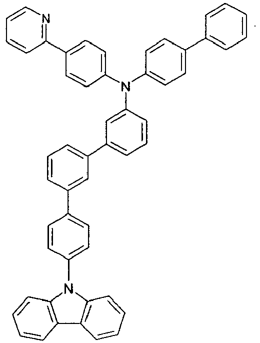 |
| 49 | 50 | 51 |
| 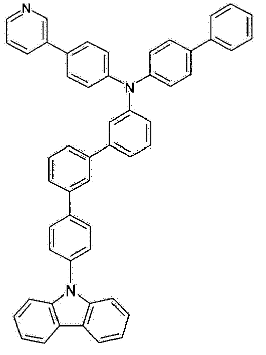 | 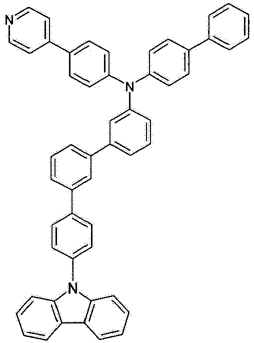 | 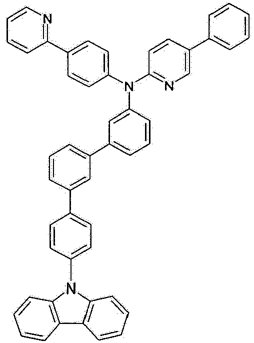 |
| 52 | 53 | 54 |
| 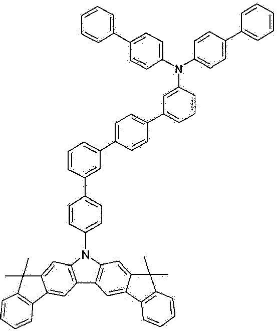 | 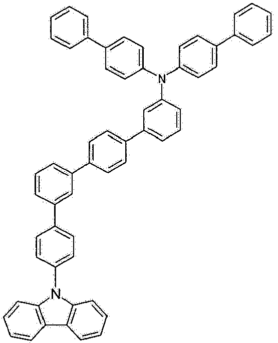 | 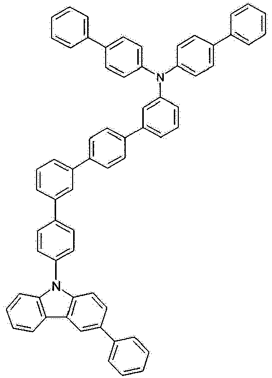 |
| 55 # | 56 # | 57 # |
| 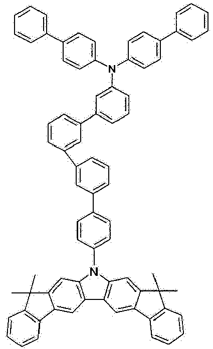 | 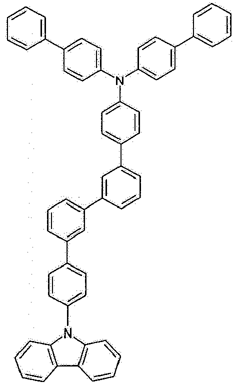 | 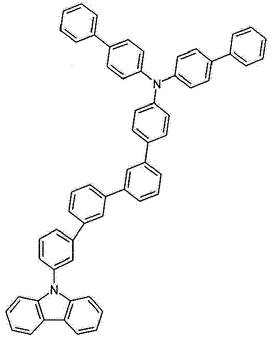 |
| 58 # | 59 # | 60 # |
| 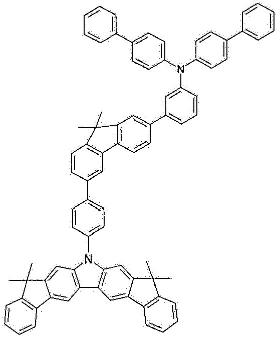 | 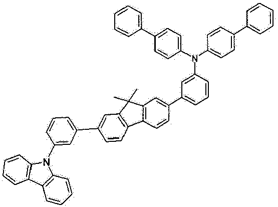 | 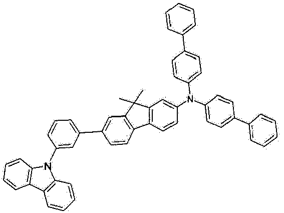 |
| 61 # | 62 # | 63 |
| 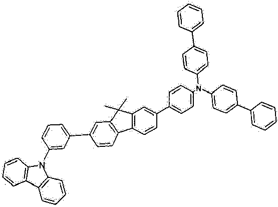 | 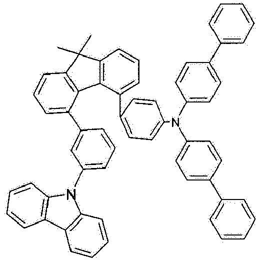 | 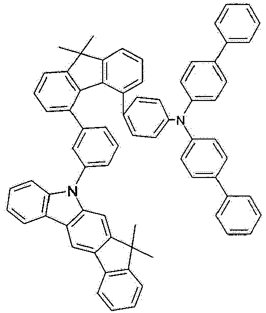 |
| 64 # | 65 # | 66 # |
| 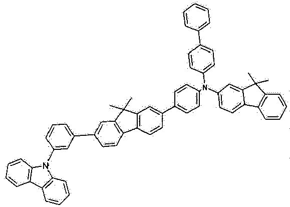 | 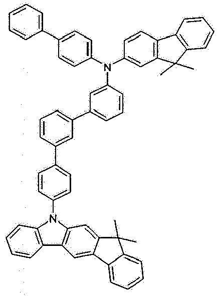 | 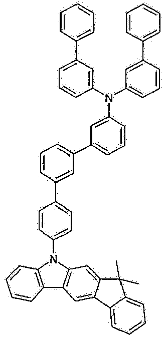 |
| 67 # | 68 | 69 |
| 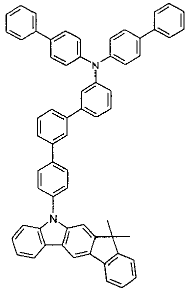 | 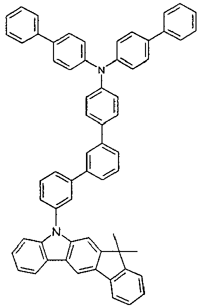 | 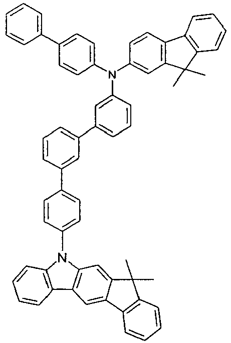 |
| 70 | 71 | 72 |

Zur Herstellung der erfindungsgemäßen Verbindungen können allgemein bekannte Verfahren der organischen Synthesechemie eingesetzt werden, beispielsweise Buchwald-Kupplung, Ullmann-Kupplung und Suzuki-Kupplung.

Im Folgenden wird ein bevorzugtes allgemein verwendbares Verfahren zur Herstellung von Verbindungen gemäß Formel (I) vorgestellt (Schema 1).

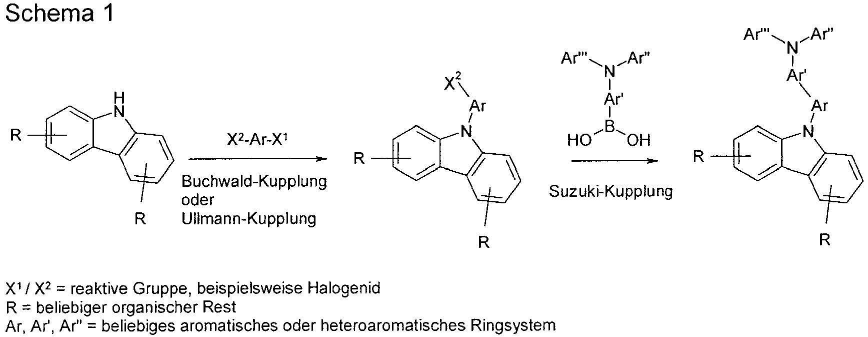

Dazu wird ausgehend von Carbazolderivaten mit freier N-H-Funktion in einer Kupplungsreaktion, bevorzugt einer Buchwald-Kupplung oder einer Ullmann-Kupplung, mit einer Arylverbindung ein N-Aryl-Carbazolderivat hergestellt. Die Carbazolderivate sind entweder kommerziell erhältlich oder können einfach hergestellt werden. Anstelle von einfachem Carbazol können auch beispielsweise Indenocarbazol oder andere Derivate von Carbazol verwendet werden.

Die Arylverbindung, mit der die Kupplungsreaktion durchgeführt wird, weist bevorzugt zwei reaktive funktionelle Gruppen auf, so dass sie in einer weiteren Kupplungsreaktion, bevorzugt in einer Suzuki-Kupplung, mit einer Arylaminoverbindung umgesetzt werden kann. Solche Arylverbindungen mit zwei reaktiven funktionellen Gruppen sind ebenfalls in vielen Fällen kommerziell erhältlich oder können einfach hergestellt werden.

Durch die Suzuki-Kupplungsreaktion kann schließlich die erfindungsgemäße Verbindung der Formel (I) erhalten werden. Gegebenfalls können sich weitere Reaktionsschritte anschließen, beispielsweise Funktionalisierungsreaktionen, um zu den endgültigen Verbindungen nach Formel (I) zu gelangen.

Das gezeigte beispielhafte Verfahren ist besonders geeignet, um erfindungsgemäße Verbindungen herzustellen. Es sind jedoch alternative Verfahren denkbar und möglicherweise in bestimmten Fällen vorzuziehen. Entsprechend kann der Fachmann das oben gezeigte Verfahren im Rahmen seines allgemeinen Fachwissens abwandeln.

Gegenstand der Erfindung ist somit weiterhin ein Verfahren zur Herstellung einer Verbindung nach Formel (I), dadurch gekennzeichnet, dass ein Carbazolderivat in einer Kupplungsreaktion mit einer Arylverbindung umgesetzt wird.

Bevorzugt ist die Kupplungsreaktion eine Buchwald-Kupplung. Weiterhin bevorzugt ist die Arylverbindung eine Verbindung mit zwei reaktiven Gruppen, von denen eine in der ersten Kupplungsreaktion reagiert, während die andere in einer zweiten, folgenden Kupplungsreaktion reagiert. Bevorzugt ist die zweite, folgende Kupplungsreaktion eine Reaktion mit einer Arylaminoverbindung, bevorzugt eine Suzuki-Kupplungsreaktion.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen gemäß Formel (I) eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung einer Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Gegenstand der Erfindung ist weiterhin eine elektronische Vorrichtung, enthaltend mindestens eine Verbindung gemäß Formel (I). Dabei ist die elektronische Vorrichtung bevorzugt ausgewählt aus den oben genannten Vorrichtungen. Besonders bevorzugt ist eine organische Elektrolumineszenzvorrichtung, enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (I) enthält. Bevorzugt ist die Verbindung der Formel (I) in einer Lochtransportschicht, einer Lochinjektionsschicht, einer Elektronenblockierschicht oder in einer emittierenden Schicht enthalten. Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die Abfolge der Schichten der organischen Elektrolumineszenzvorrichtung ist bevorzugt die folgende:
Anode-Lochinjektionsschicht-Lochtransportschicht-emittierende Schicht-Elektronentransportschicht-Elektroneninjektionsschicht-Kathode.

Dabei soll erneut darauf hingewiesen werden, dass nicht alle der genannten Schichten vorhanden sein müssen, und/oder dass zusätzlich weitere Schichten vorhanden sein können.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei bevorzugt mindestens eine dieser Schichten mindestens eine Verbindung gemäß Formel (I) enthält und wobei die drei Schichten blaue, grüne und orange oder rote Emission zeigen (für den prinzipiellen Aufbau siehe z. B. WO 2005/011013). Alternativ und/oder zusätzlich können die erfindungsgemäßen Verbindungen auch in der Lochtransportschicht oder in einer anderen Schicht vorhanden sein.

Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist erfindungsgemäß bevorzugt, wenn die Verbindung gemäß Formel (I) in einer elektronischen Vorrichtung enthaltend einen oder mehrere phosphoreszierende Dotanden eingesetzt wird. Dabei kann die Verbindung in unterschiedlichen Schichten, bevorzugt in einer Lochtransportschicht, einer Lochinjektionsschicht, einer Elektronenblockierschicht oder in einer emittierenden Schicht, verwendet werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen Emitter können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (I) in organischen Elektrolumineszenzvorrichtungen einsetzen.

Explizite Beispiele für geeignete phosphoreszierende Emitterverbindungen können einer folgenden Tabelle entnommen werden.

Die Verbindung gemäß Formel (I) kann aber auch erfindungsgemäß in einer elektronischen Vorrichtung enthaltend einen oder mehrere fluoreszierende Dotanden eingesetzt werden.

In einer bevorzugten Ausführungsform der Erfindung werden die Verbindungen gemäß Formel (I) als Lochtransportmaterial eingesetzt. Die Verbindungen werden dann bevorzugt in einer Lochtransportschicht, einer Elektronenblockierschicht oder einer Lochinjektionsschicht eingesetzt.

Eine Lochtransportschicht gemäß der vorliegenden Anmeldung ist eine Schicht mit lochtransportierender Funktion, welche sich zwischen Anode und emittierender Schicht befindet.

Lochinjektionsschichten und Elektronenblockierschichten werden im Sinne der vorliegenden Anmeldung als spezielle Ausführungsformen von Lochtransportschichten verstanden. Eine Lochinjektionsschicht ist dabei im Fall von mehreren Lochtransportschichten zwischen Anode und emittierender Schicht eine Lochtransportschicht, welche sich direkt an die Anode anschließt oder nur durch eine einzelne Beschichtung der Anode von ihr getrennt ist. Eine Elektronenblockierschicht ist im Fall von mehreren Lochtransportschichten zwischen Anode und emittierender Schicht diejenige Lochtransportschicht, welche sich direkt anodenseitig an die emittierende Schicht anschließt.

Wird die Verbindung gemäß Formel (I) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (I) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 und WO 2012/095143 offenbarten Verbindungen.

In einer weiteren bevorzugten Ausführungsform der Erfindung wird die Verbindung gemäß Formel (I) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

In einer weiteren Ausführungsform der vorliegenden Erfindung werden die Verbindungen der Formel (I) als Matrixmaterial in Kombination mit einem oder mehreren Dotanden, vorzugsweise phosphoreszierenden Dotanden, eingesetzt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß Formel (I) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere Dotanden umfassen, bevorzugt einen oder mehrere phosphoreszierende Dotanden. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im mixed-Matrix-System eingesetzt wird.

Bevorzugte phosphoreszierende Dotanden zur Verwendung in Mixed-Matrix-Systemen sind die obenstehend und in die in einer folgenden Tabelle aufgeführten phosphoreszierenden Dotanden.

Im Folgenden werden in den erfindungsgemäßen Vorrichtungen bevorzugt eingesetzte Materialien aufgeführt, geordnet nach ihrer Verwendung und Funktion.

Explizite Beispiele für phosphoreszierende Dotanden sind in der folgenden Tabelle aufgeführt.

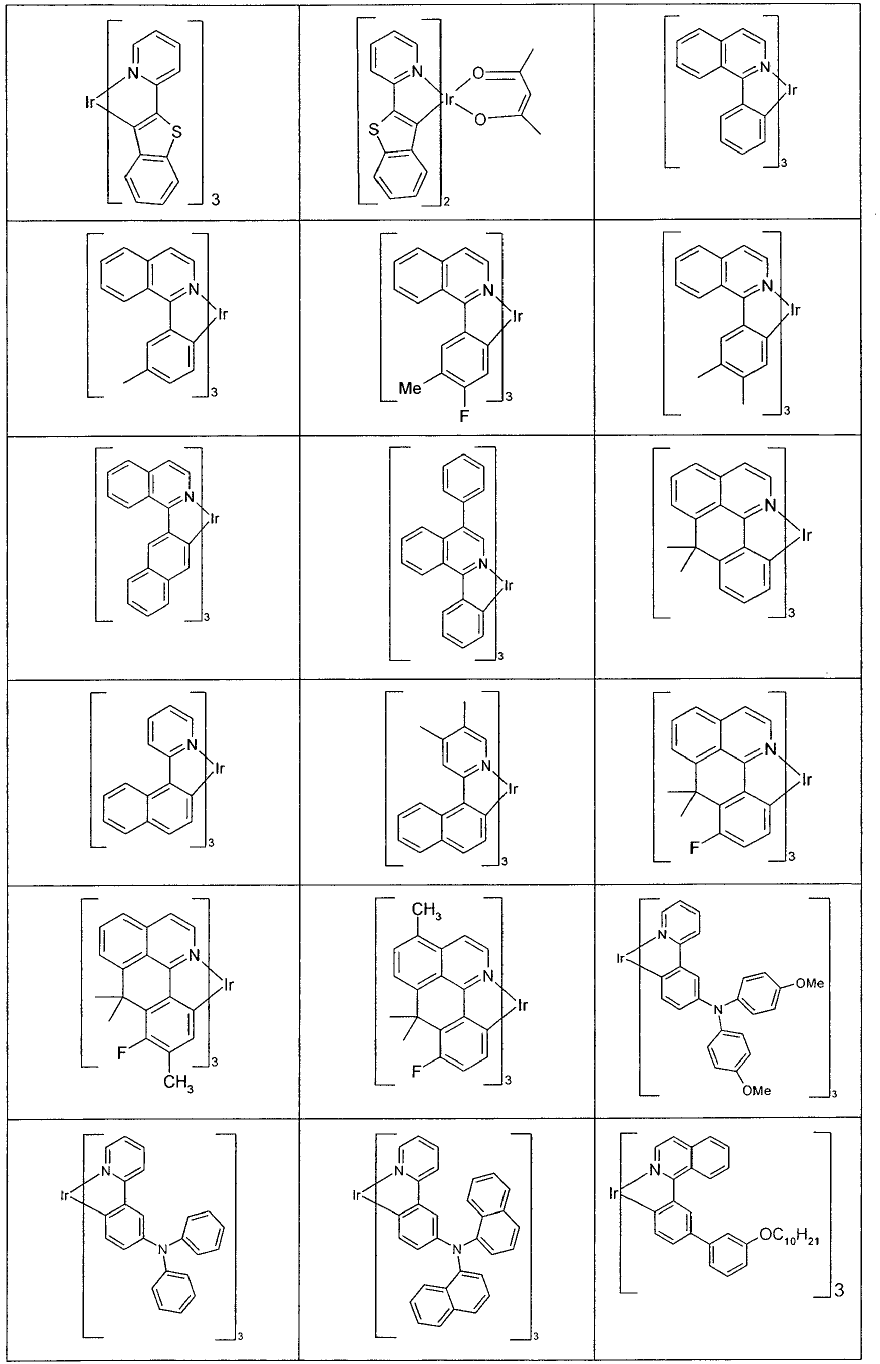

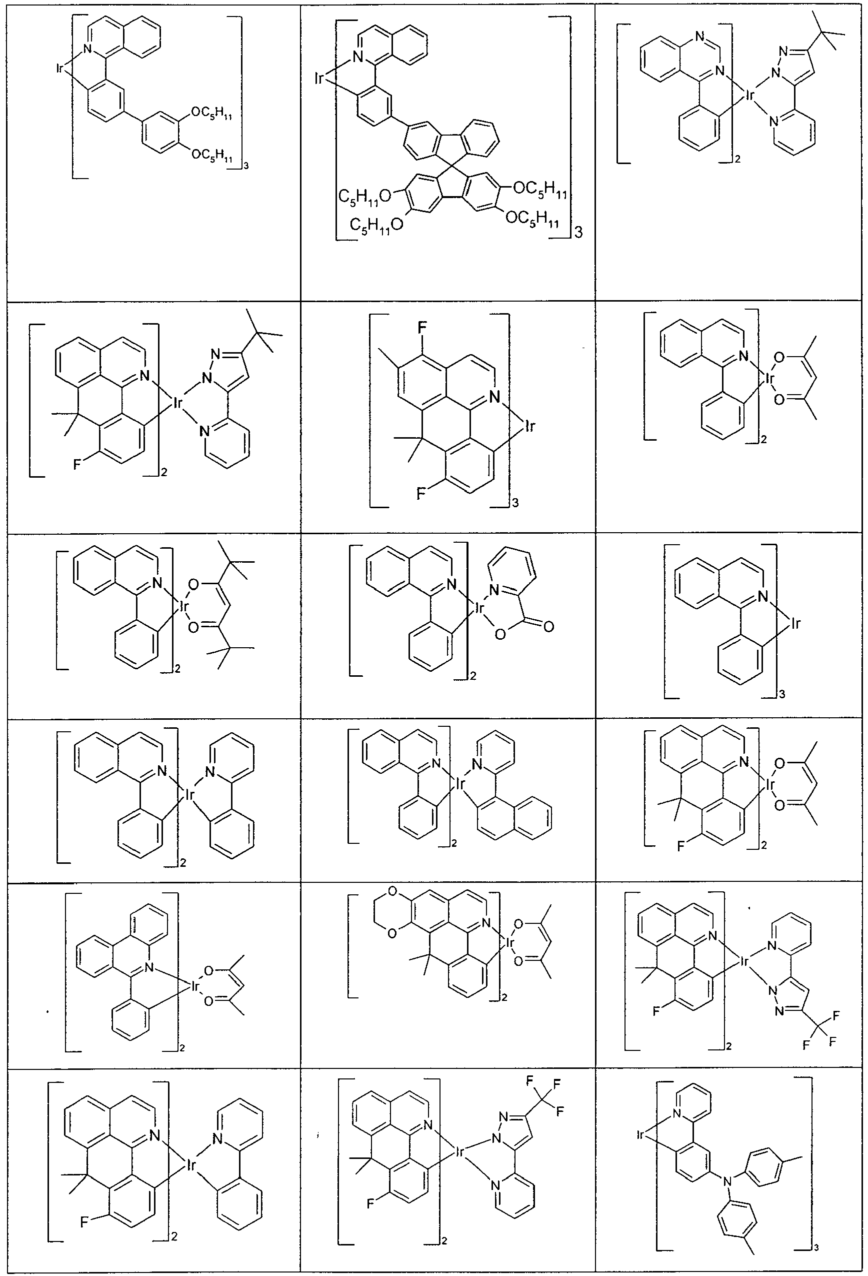

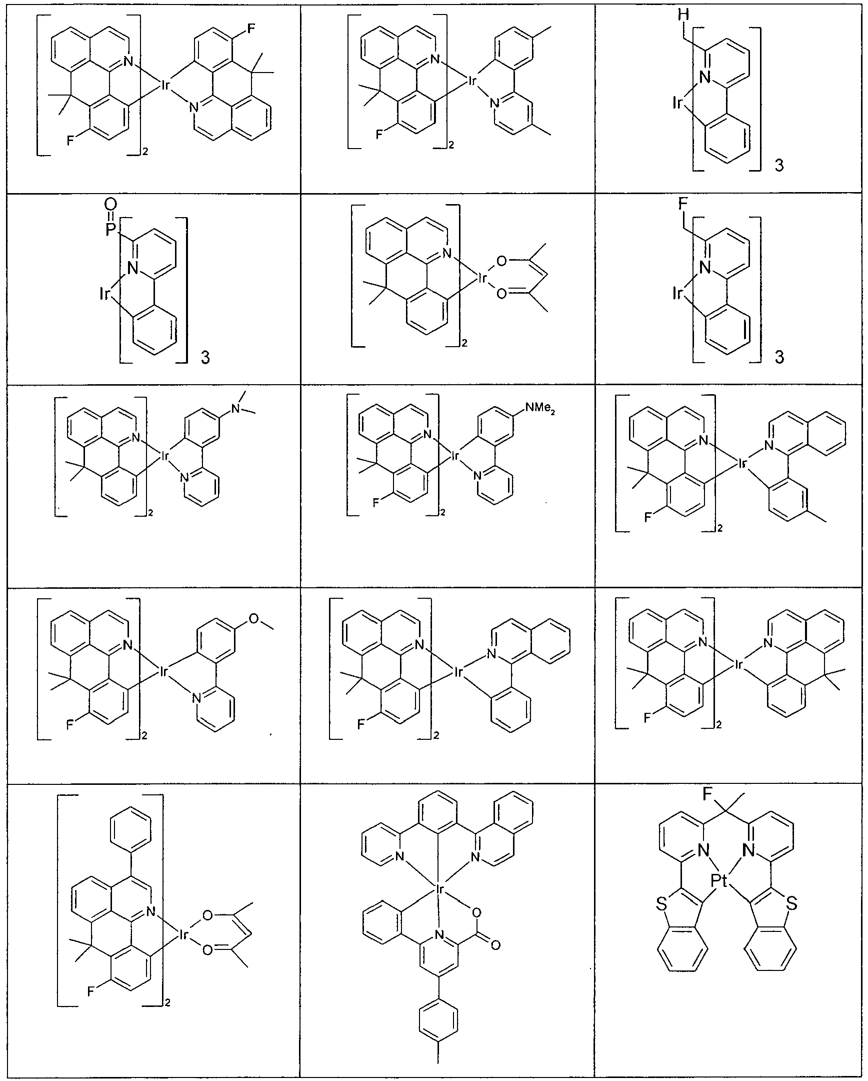

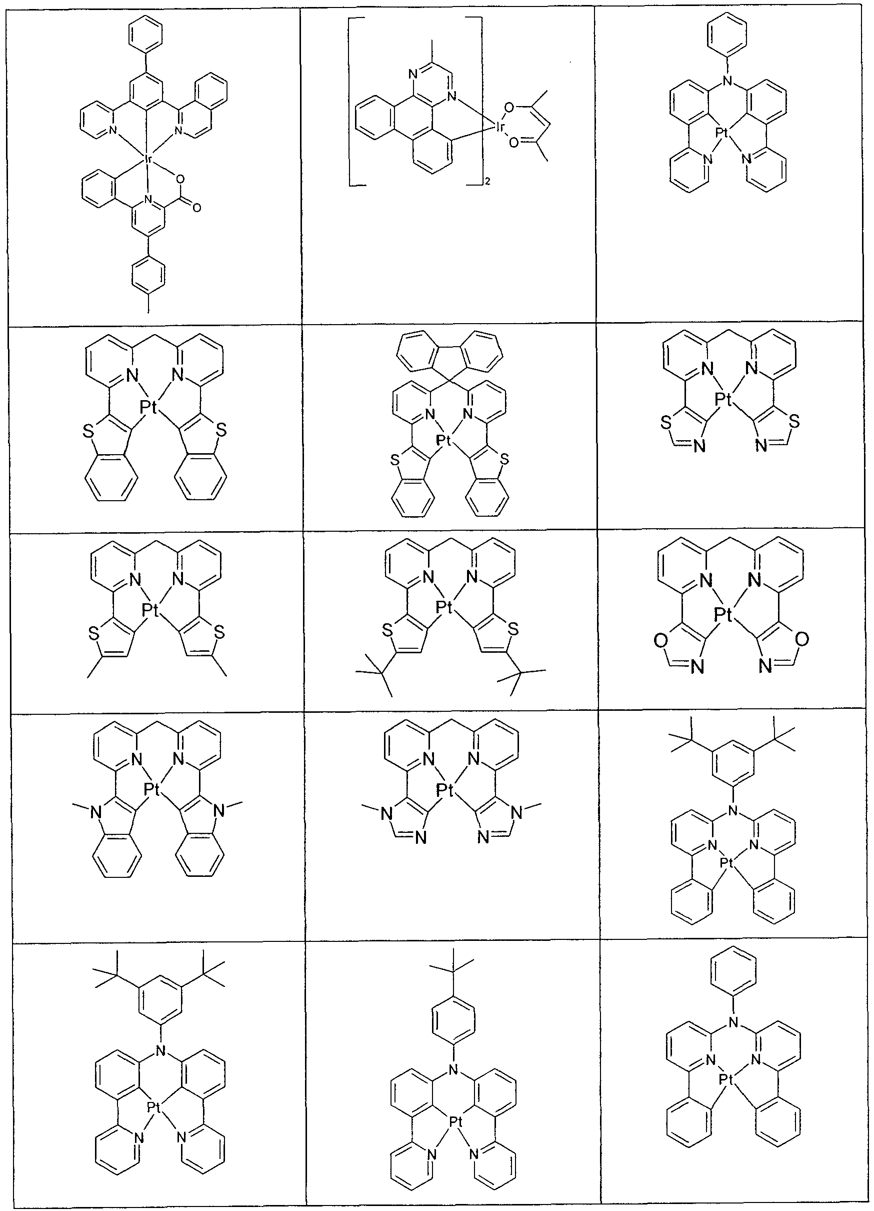

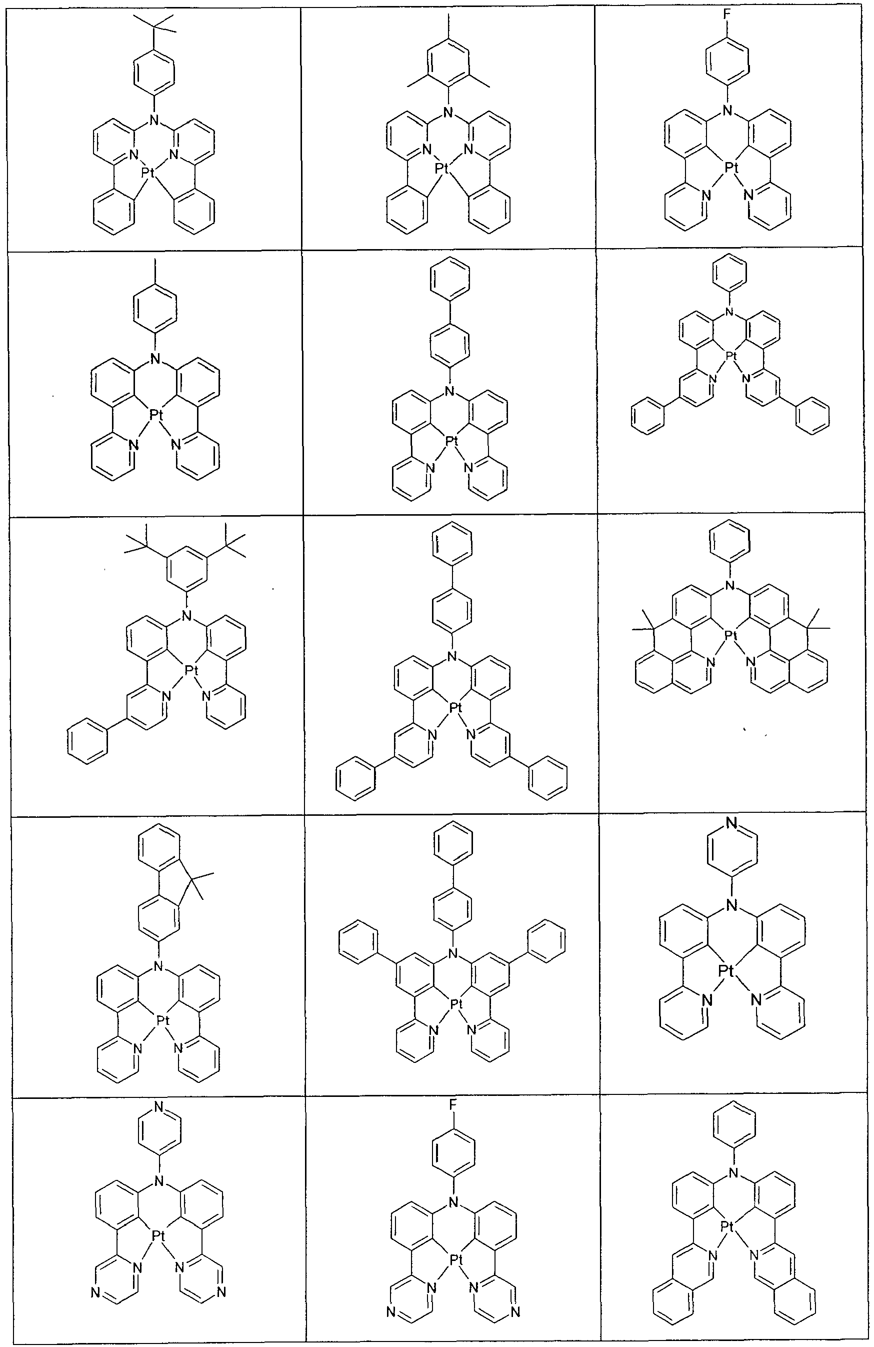

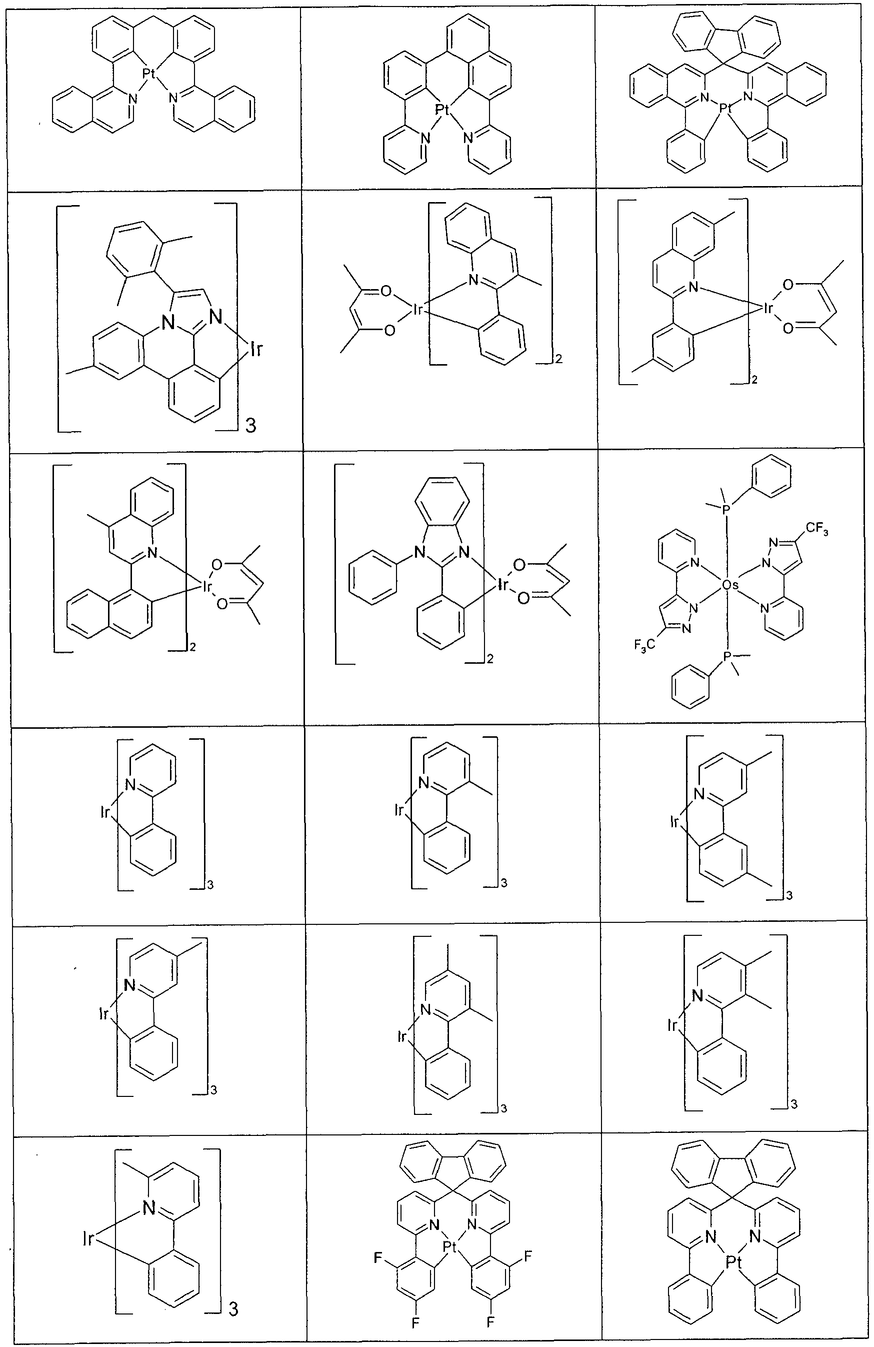

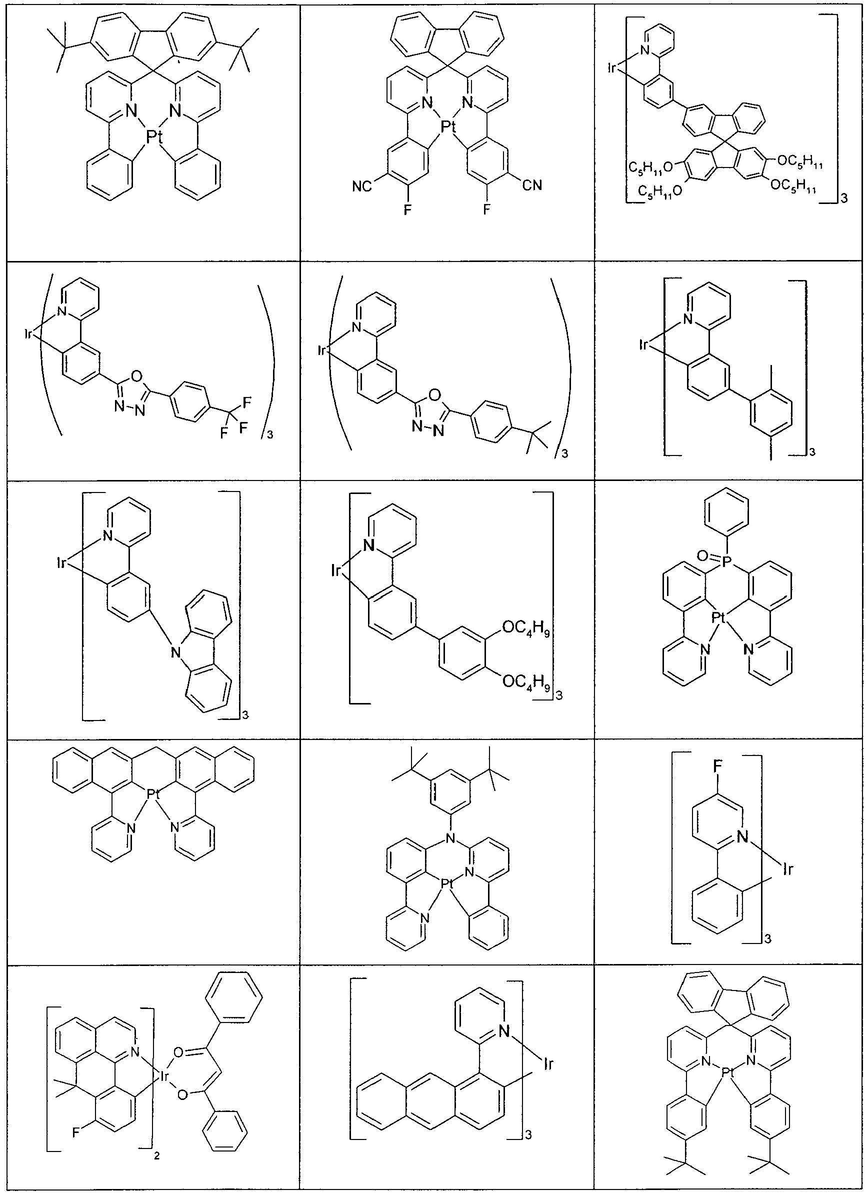

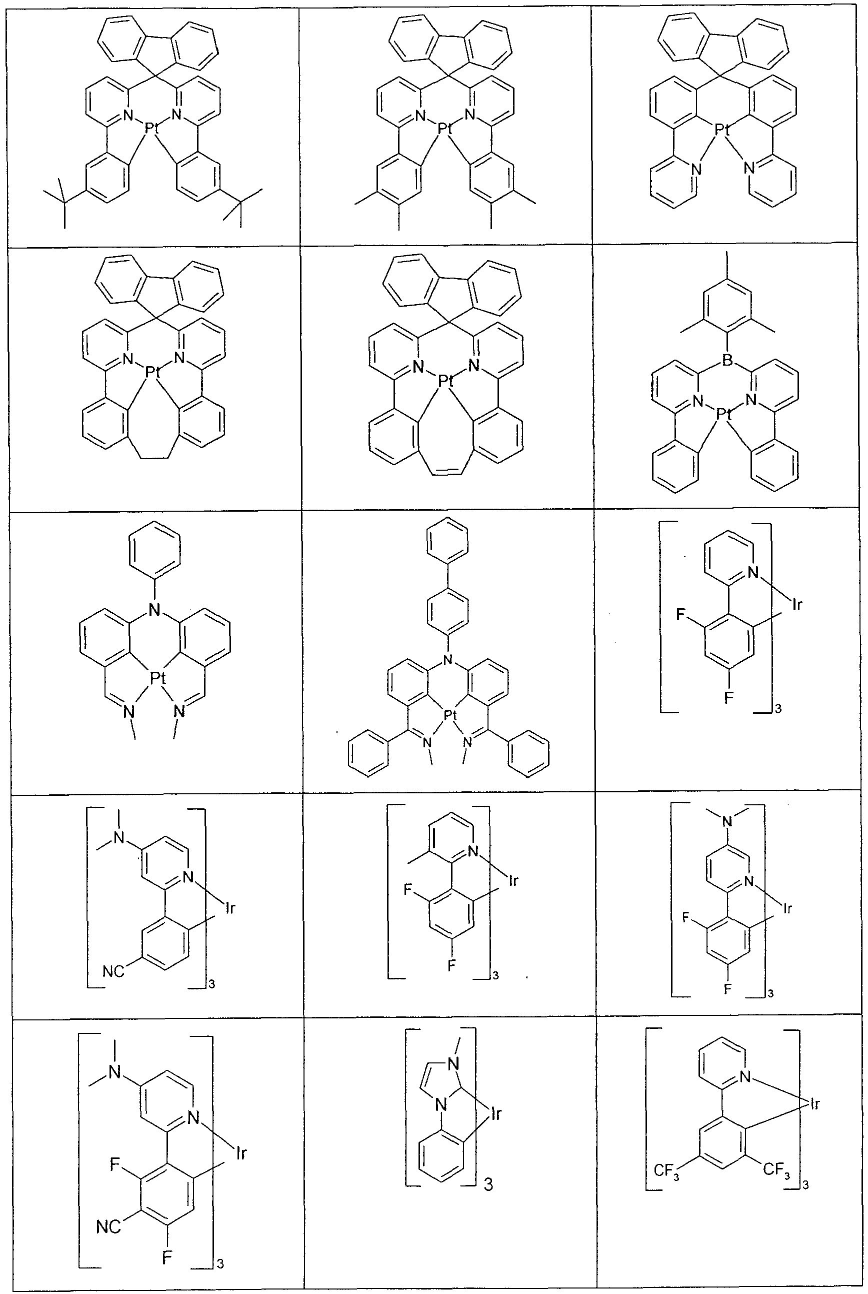

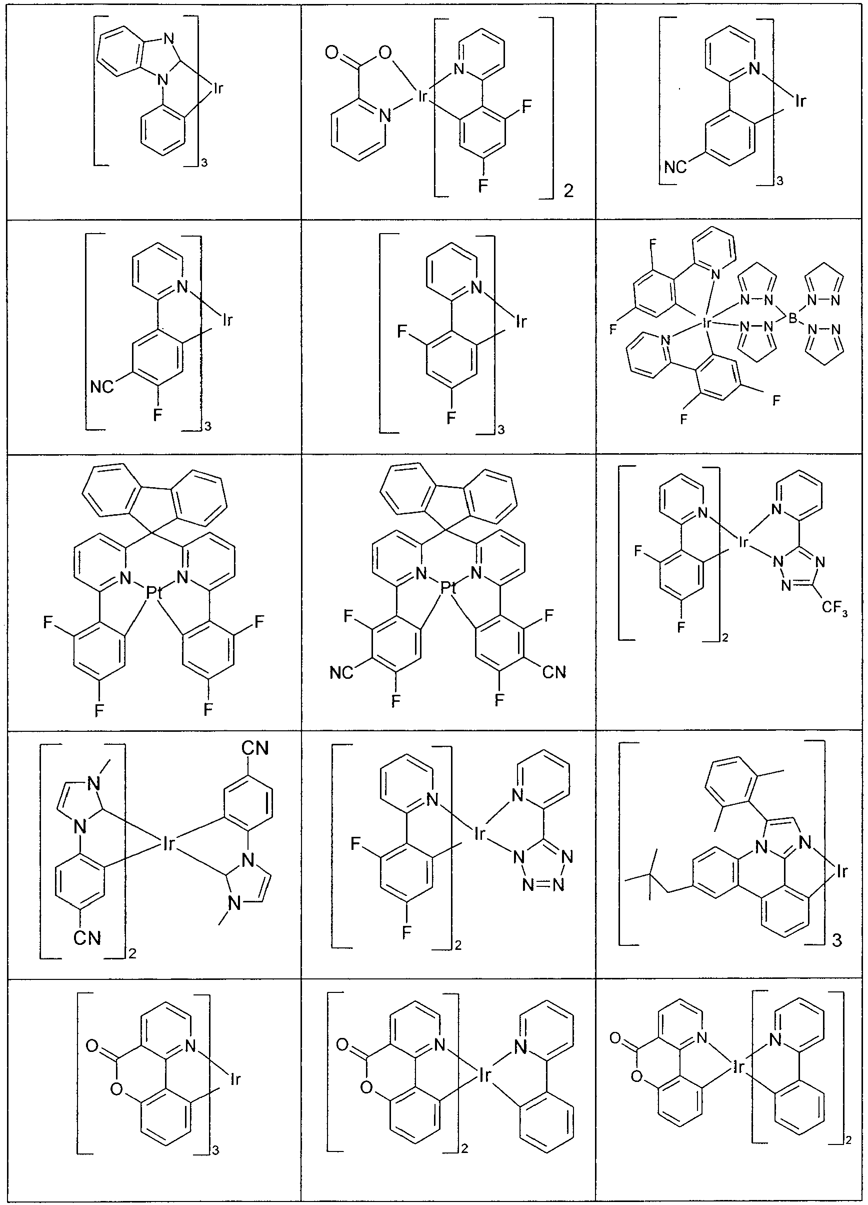

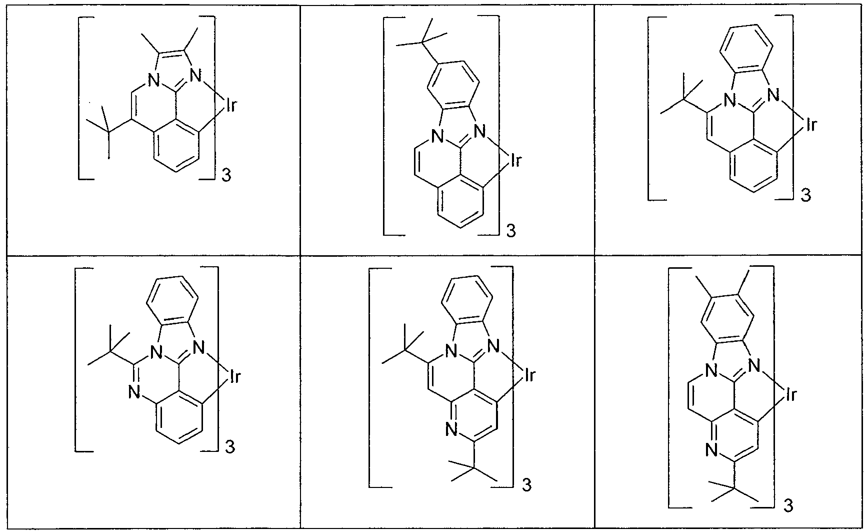

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Emitter sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und der noch nicht offengelegten EP 12004426.8 offenbarten Pyren-Arylamine. Ebenfalls bevorzugt sind die in der noch nicht offengelegten EP 12006239.3 offenbarten Benzoindenofluoren-Amine und die in der noch nicht offengelegten EP 13000012.8 offenbarten Benzofluoren-Amine.

Als Matrixmaterialien, bevorzugt für fluoreszierende Emitter, kommen neben den erfindungsgemäßen Verbindungen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, sowie die in EP 1749809, EP 1905754 und US 2012/0187826 offenbarten Pyren-Verbindungen. Bevorzugte Matrixmaterialien für phosphoreszierende Emitter sind neben den erfindungsgemäßen Verbindungen aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Geeignete Materialien sind beispielsweise die in der folgenden Tabelle aufgeführten Materialien. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Bevorzugt sind als Lochtransportmaterialien, die in einer Lochtransport-, Lochinjektions- oder Elektronenblockierschicht in der erfindungsgemäßen Elektrolumineszenzvorrichtung verwendet werden können, Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder der noch nicht offengelegten EP 12000929.5), Fluoren-Amine (z. B. gemäß den noch nicht offengelegten Anmeldungen EP 12005369.9, EP 12005370.7 und EP 12005371.5), Spiro-Dibenzopyran-Amine (z. B. gemäß WO 2013/083216) und Dihydroacridin-Derivate (z. B. gemäß WO 2012/150001). Insbesondere die erfindungsgemäßen Verbindungen können als Lochtransportmaterialien verwendet werden.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder AI, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Weiterhin können die Verbindungen nach Formel (I) so gewählt sein, dass sie in einer aus Lösung aufgebrachten Schicht fixiert werden können, beispielsweise durch Vernetzung zu einem Polymernetzwerk. Das Polymernetzwerk ist anschließend in den beim Auftragen auf Lösung gängigerweise verwendeten Lösungsmitteln kaum oder gar nicht mehr löslich. Auf diese Weise können mehrere Schichten aus Lösung aufgebracht werden, ohne dass beim Auftragen einer folgenden Schicht aus Lösung die vorangegangene Schicht erneut gelöst wird und dabei eine Vermischung erfolgt. Ein solches Verfahren ist beispielsweise in EP 0637899 und US 6107452 allgemein offenbart. Damit die Verbindungen der Formel (I) vernetzbar sind, ist es bevorzugt, dass sie mindestens eine, bevorzugt mindestens zwei vernetzbare Gruppen aufweisen. Vernetzbare Gruppe im Sinne der vorliegenden Anmeldung bedeutet eine funktionelle Gruppe, die in der Lage ist, eine Reaktion, vorzugsweise eine Polymerisationsreaktion, einzugehen und so eine unlösliche Verbindung zu bilden. Bei der vernetzbaren Gruppe handelt es sich somit bevorzugt um eine polymerisierbare Gruppe. Dabei erhält man als Ergebnis der Reaktion der vernetzbaren Gruppe eine entsprechende vernetzte Verbindung und somit eine schwerlösliche oder unlösliche Schicht. Die Vernetzungsreaktion kann beispielsweise durch Wärme oder durch UV-, Mikrowellen-, Röntgen- oder Elektronenstrahlung unterstützt werden, gegebenenfalls in Gegenwart eines Initiators. Bevorzugte vernetzbare Gruppen sind chemische Gruppen enthaltend endständige oder cyclische Alkenyl- bzw. endständige Alkinylgruppen, Oxetane, Oxirane oder Silane. Besonders bevorzugte vernetzbare Gruppen sind die in WO 2013/007348 offenbarten vernetzbaren Gruppen.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

### A) Synthesebeispiele

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht gehandhabt. Die Lösungsmittel und Reagenzien können z. B. von Sigma-ALDRICH bzw. ABCR bezogen werden. Die Angaben in eckigen Klammern zu literaturbekannten chemischen Verbindungen beziehen sich auf die CAS-Nummer.

### Beispiel 1: 9-(3'-Chlor-biphenyl-3-yl)-9H-carbazol

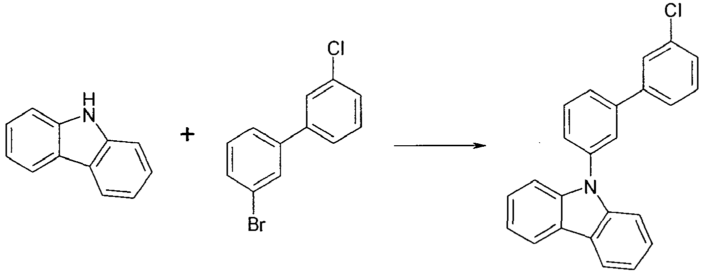

8,36 g (50 mmol) Carbazol und 14,72 g (55 mmol) 3-Bromo-3'-chloro-biphenyl werden in Toluol gelöst und via Schutzgaseinleitung entgast. Anschließend wird mit 4,90 mL (4,9 mmol, 1 M Lösung in Toluol) Tri-tert-butylphosphin, 633,70 mg (2,82 mmol) Pd(OAc)₂ und 10,21 g (105,87 mmol) t-BuONa versetzt. Die Feststoffe werden zuvor entgast, die Reaktionsmischung wird nachentgast und anschließend unter Rückfluss für 12 h gerührt. Die warme Reaktionslösung wird über Alox B (Aktivitätsstufe 1) filtriert, mit Wasser gewaschen, getrocknet und eingeengt. Der Rückstand wird aus Toluol umkristallisiert. Die Ausbeute beträgt 15,92 g (45 mmol), entsprechend 90 % der Theorie.

Analog können folgende Verbindungen erhalten werden:

| | Edukt 1 | Edukt 2 | Produkt | Ausbeute |
|---|---|---|---|---|
| 1a | 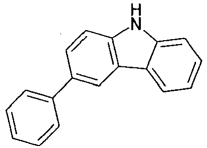 | 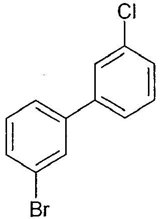 | 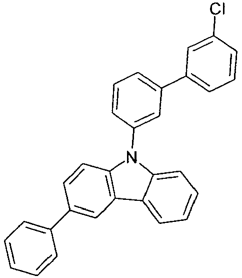 | 79% |
| | [103012-26-6] | [844856-42-4] | | |
| 1b | 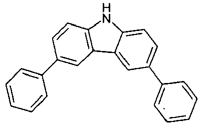 | 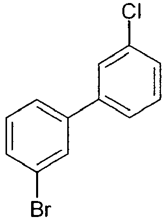 | 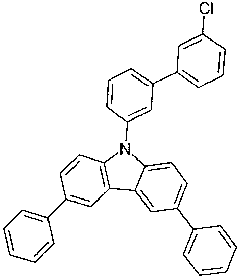 | 85% |
| | [56525-79-2] | | | |
| | | [844856-42-4] | | |
| 1c | 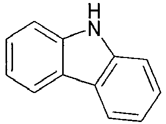 | 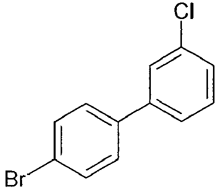 | 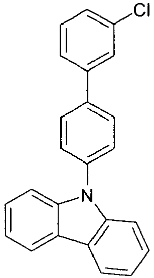 | 75% |
| | **[86-74-8]** | [91354-09-5] | | |
| 1d | 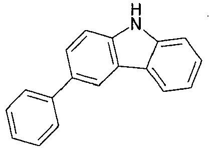 | 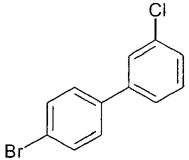 | 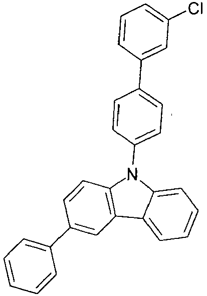 | 71% |
| | [103012-26-6] | [91354-09-5] | | |
| 1e | 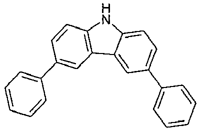 | 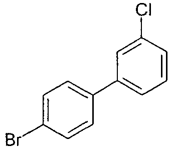 | 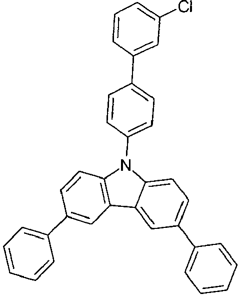 | 82% |
| | [56525-79-2] | [91354-09-5] | | |
| 1f | 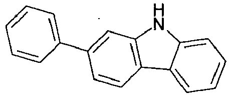 | 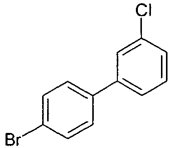 | 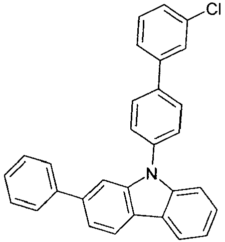 | 78% |
| | [88590-00-5] | [91354-09-5] | | |
| 1g | 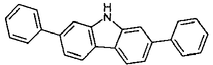 | 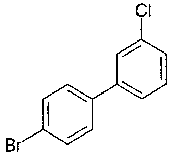 | 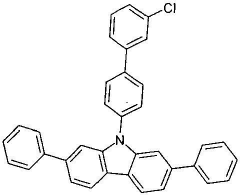 | 70% |
| | [42448-04-4] | | | |
| | | [91354-09-5] | | |
| 1h | 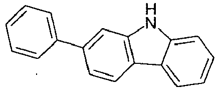 | 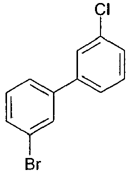 | 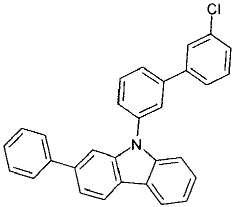 | 78% |
| | [88590-00-5] | [844856-42-4] | | |
| 1i | 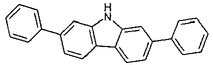 | 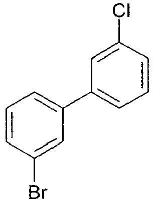 | 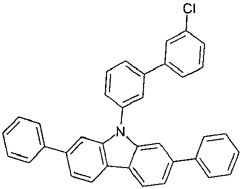 | |
| | [42448-04-4] | | | |
| | | [844856-42-4] | | |
| 1j | 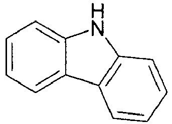 | 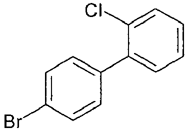 | 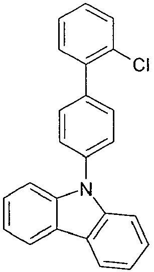 | 72% |
| | **[86-74-8]** | [39802-88-5] | | |
| 1k | 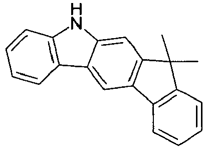 | 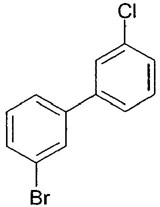 | 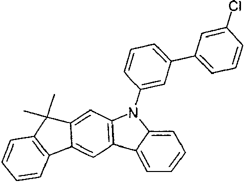 | 79% |
| | [1257220-47-5] | [844856-42-4] | | |
| 1l | 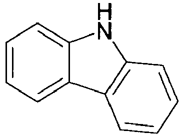 | 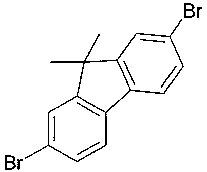 | 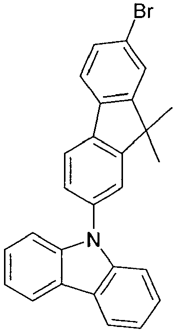 | 55% |
| | [86-74-8] | [28320-32-3] | | |
| 1m | 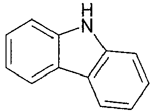 | 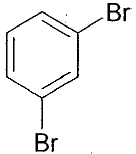 | 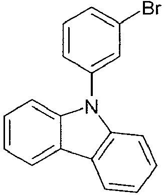 | 65% |
| | [ [86-74-8 ] | **[108-36-1]** | | |

### Beispiel 2: Bis-biphenyl-4-yl-(3-carbazol-9-yl-[1,1';3',1"]terphenyl-4"-yl)-amin

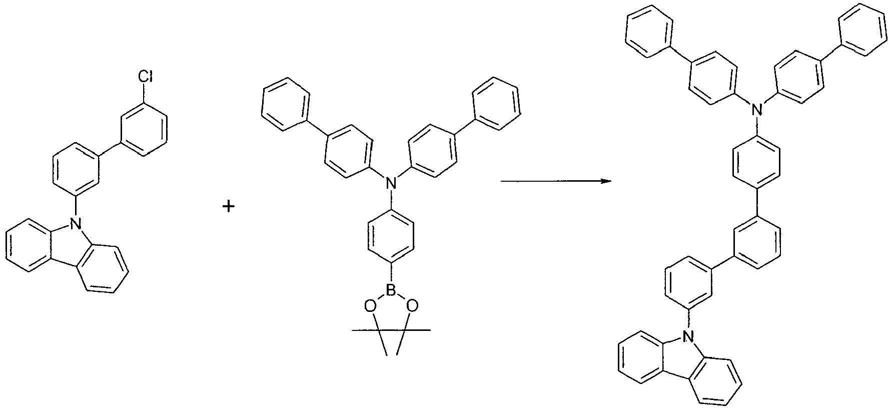

36,61 g (70 mmol) Bis-biphenyl-4-yl-[4-(4,4,5,5-tetramethyl-[1,3,2]dioxaborolan-2-yl)-phenyl]-amin, 23,00 g (65 mmol) 9-(3'-Chlor-biphenyl-3-yl)-9H-carbazol und 78,9 ml (158 mmol) Na₂CO₃ (2M-Lösung) werden in 120 mL Ethanol und 120 mL Toluol suspendiert. Zu dieser Suspension werden 4,06 g (3,51 mmol) Pd(PPh₃)₄ gegeben, und die Reaktionsmischung wird 16 h unter Rückfluss erhitzt. Nach Erkalten wird die Mischung mit Dichloromethan versetzt, die organische Phase abgetrennt, über MgSO₄ getrocknet und das Lösungsmittel im Vakuum entfernt. Danach wird das Rohprodukt über Kieselgel mit Heptan/Essigsäureester (20:1) chromatographisch gereinigt. Der Rückstand wird aus Toluol umkristallisiert. Die Ausbeute beträgt 40,75 g (57 mmol), entsprechend 87,9 % der Theorie. Der Zielverbindung wird abschließend im Hochvakuum (p = 5 x 10⁻⁶ mbar) sublimiert. Die Reinheit beträgt 99.9%.

Analog können folgende Verbindungen erhalten werden:

| | Edukt 1 | Edukt 2 | Produkt | Ausbeute |
|---|---|---|---|---|
| 2a | 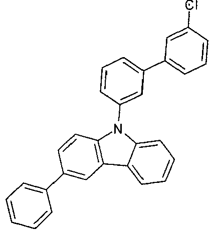 | 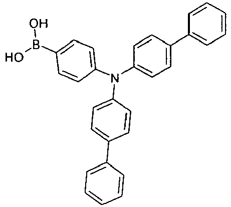 | 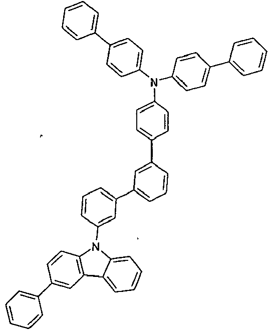 | 79% |
| | | [943836-24-6] | | |
| 2b | 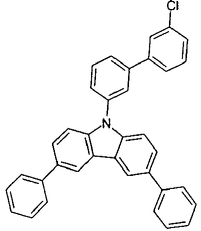 | 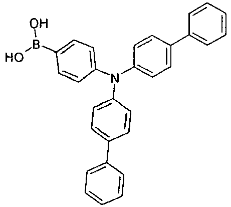 | 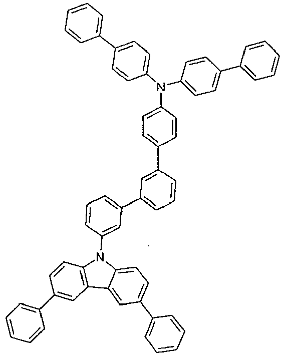 | 85% |
| | | [943836-24-6] | | |
| 2c | 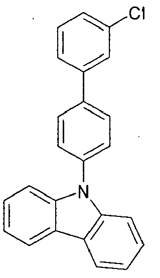 | 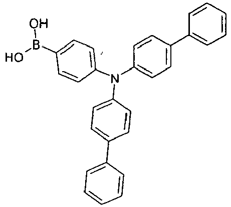 | 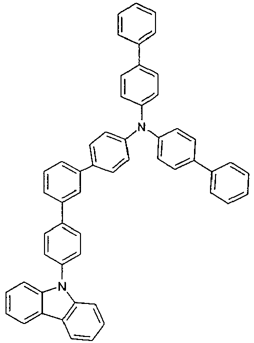 | 75% |
| | | [943836-24-6] | | |
| 2d | 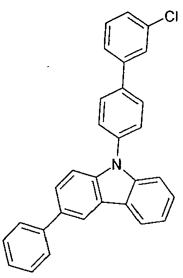 | 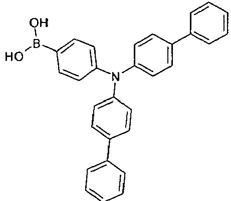 | 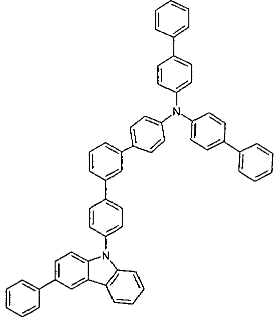 | 71% |
| | | [943836-24-6] | | |
| 2e | 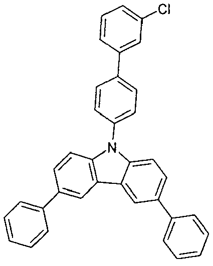 | 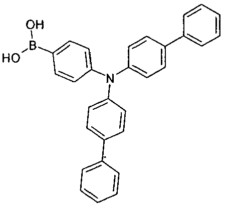 | 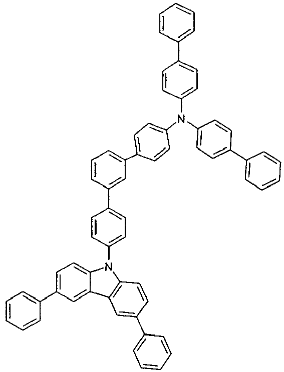 | 82% |
| | | [943836-24-6] | | |
| 2f | 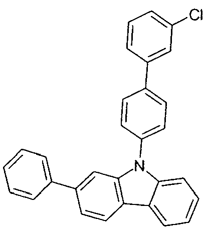 | 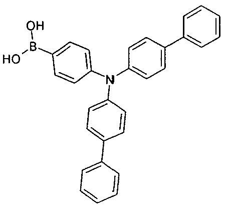 | 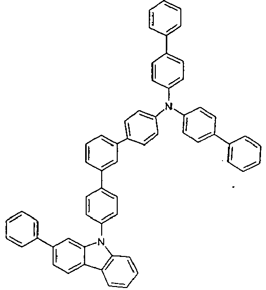 | 78% |
| | | [943836-24-6] | | |
| 2g | 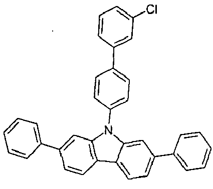 | 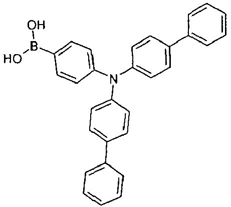 | 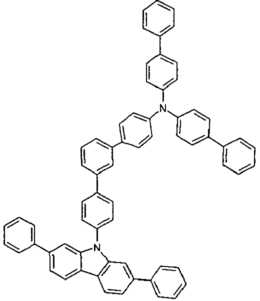 | 70% |
| | | [943836-24-6 ] | | |
| 2h | 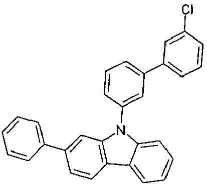 | 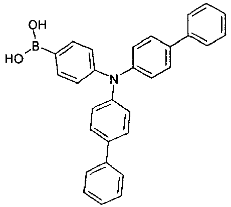 | 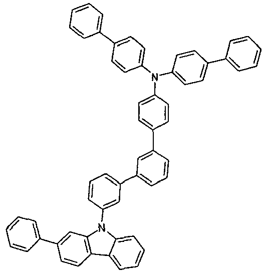 | 78% |
| | | [943836-24-6] | | |
| 2i | 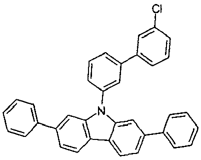 | 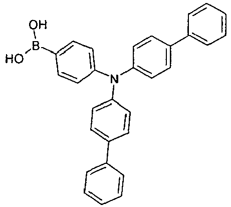 | 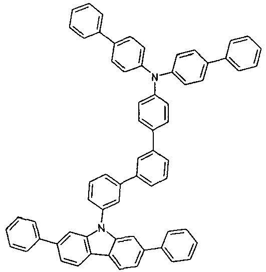 | 83% |
| | | [943836-24-6] | | |
| 2k | 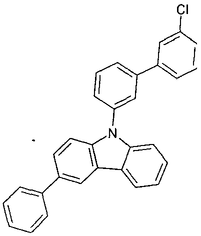 | 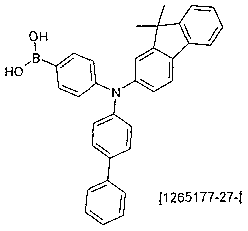 | 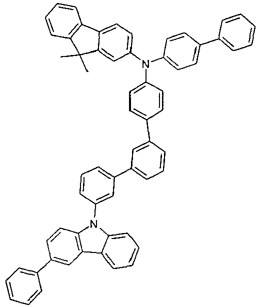 | 79% |
| 2l | 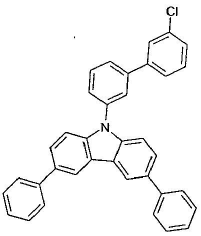 | 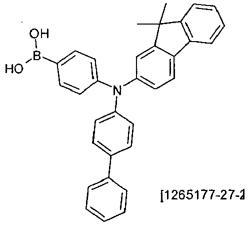 | 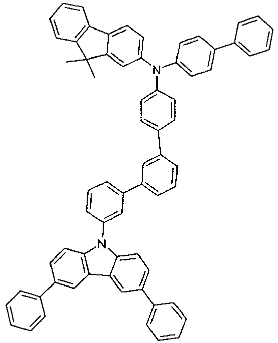 | 85% |
| 2m | 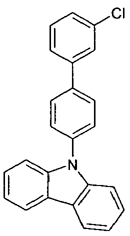 | 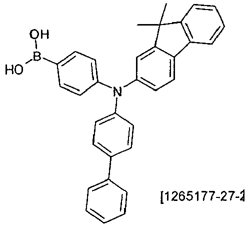 | 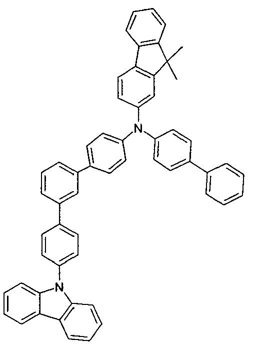 | 75% |
| 2n | 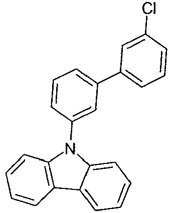 | 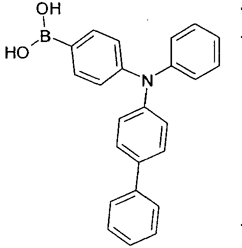 | 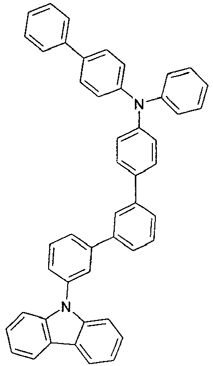 | 79% |
| 2o | 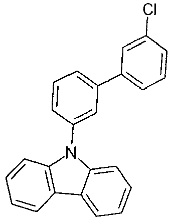 | 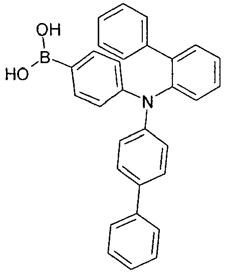 | 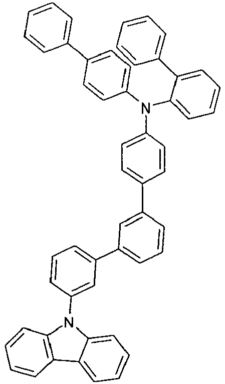 | 82% |
| 2p | 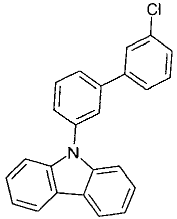 | 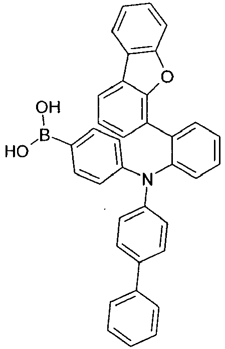 | 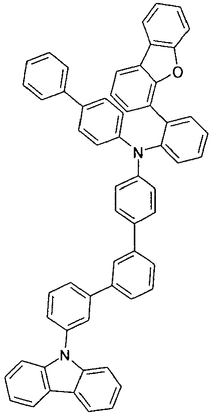 | 74% |
| 2r | 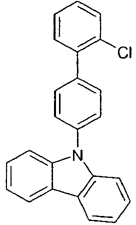 | 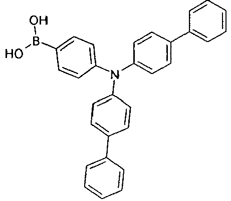 | 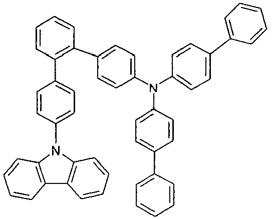 | 70% |
| | | [943836-24-6] | | |
| 2m | 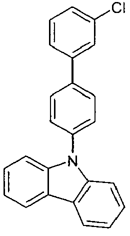 | 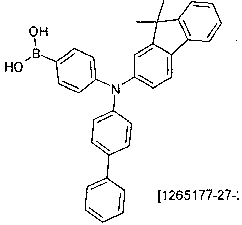 | 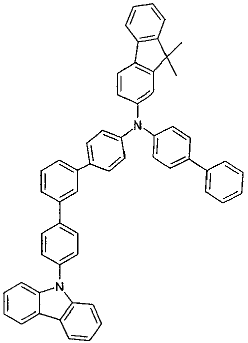 | 75% |
| 2s | 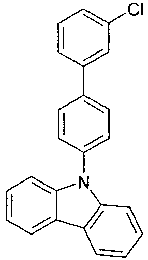 | 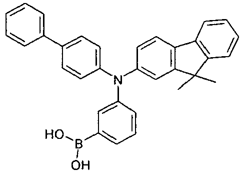 | 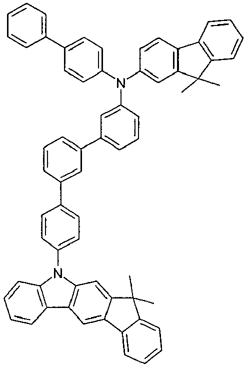 | 68% |
| 2t | 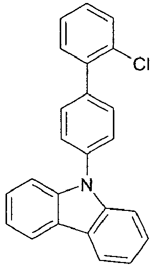 | 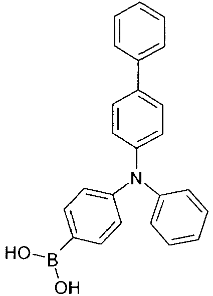 | 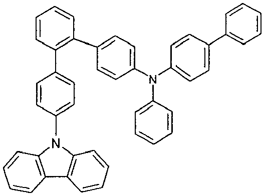 | 61% |
| | | [1084334-86-0] | | |
| 2u | 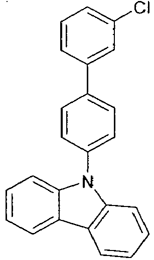 | 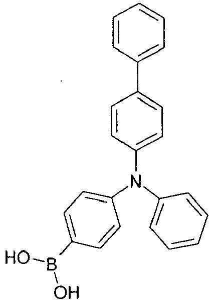 | 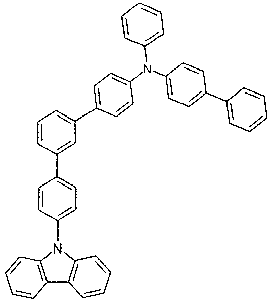 | 74% |
| | | [1084334-86-0] | | |
| 2v | 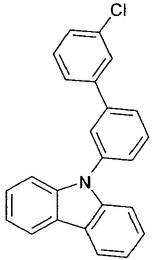 | 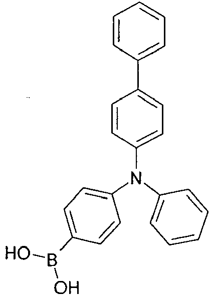 | 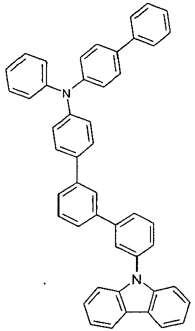 | 75% |
| | | [1084334-86-0] | | |
| 2w | 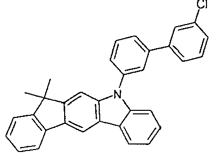 | 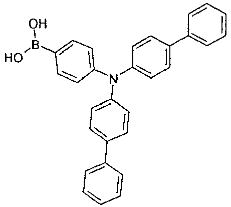 | 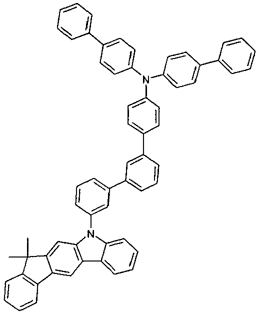 | 68% |
| | | [943836-24-6 ] | | |
| 2y | 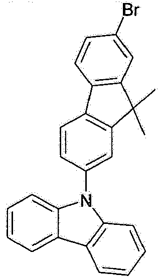 | 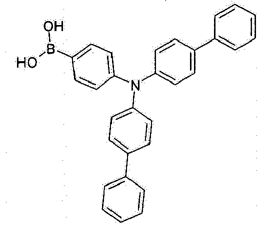 | 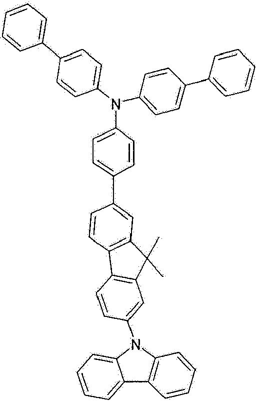 | 68% |
| | | [943836-24-6] | Nicht anspruchsgemäß | |
| 2z | 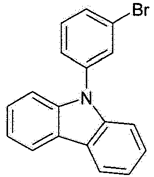 | 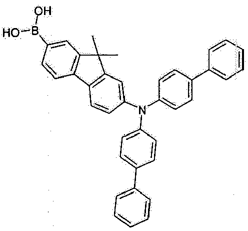 | 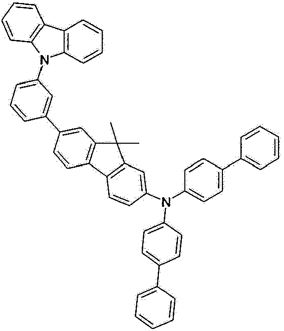 | 70 |
| | | [1265177-39-6] | | |

### B) Device-Beispiele

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden anmeldungsgemäßen Beispielen E1 bis E9 und in den Referenzbeispielen V1-V6 werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben prinzipiell folgenden Schichtaufbau: Substrat / p-dotierte Lochtransportschicht (HTL1) / Lochtransportschicht (HTL2) / p-dotierte Lochtransportschicht (HTL3) / Lochtransportschicht (HTL4) / Emissionsschicht (EML) / Elektronentransportschicht (ETL) / Elektroneninjektionsschicht (EIL) und abschließend eine Kathode. Die Kathode wird durch eine 100 nm dicke Aluminiumschicht gebildet. Die zur Herstellung der OLEDs benötigten Materialien sind in Tabelle 1 gezeigt, die genauen Aufbauten der hergestellten Vorrichtungen in Tabelle 2.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:SEB1(5%) bedeutet hierbei, dass das Material H1 in einem Volumenanteil von 95% und SEB1 in einem Anteil von 5% in der Schicht vorliegt. Analog können auch die Elektronentransportschichten oder die Lochtransportschichten aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik, sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe EQE @ 10 mA/cm² bezeichnet die externe Quanteneffizienz bei einer Stromdichte von 10mA/cm². LD80 @ 50 mA/cm² ist die Lebensdauer, bis die OLED bei einer Starthelligkeit bei konstantem Strom von 50mA/cm² auf 80 % der Anfangsintensität abgefallen ist.

| Tabelle 1: Strukturen der verwendeten Materialien | | |
|---|---|---|
| 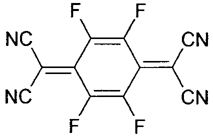 | 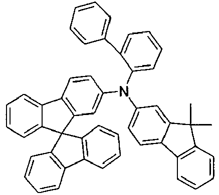 | 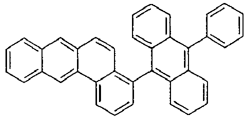 |
| F4TCNQ | ILH | H1 |
| 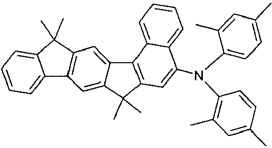 | 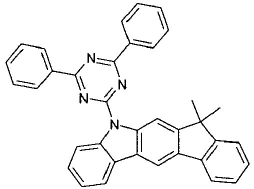 | 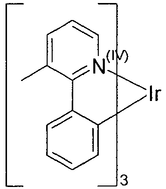 |
| SEB | H2 | TEG |
| 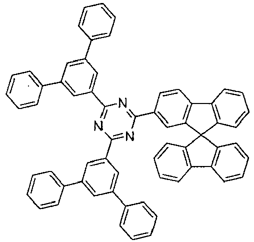 | 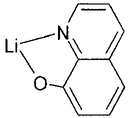 | |
| ETM | LiQ | |
| 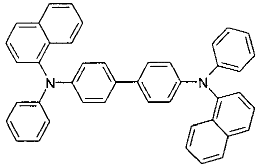 | 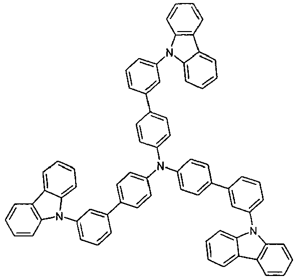 | 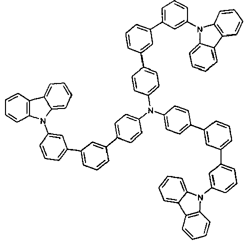 |
| NPB | HTMV1 | HTMV2 |
| 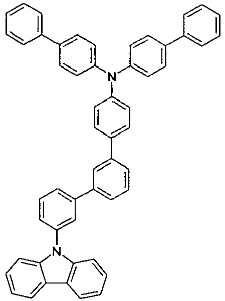 | 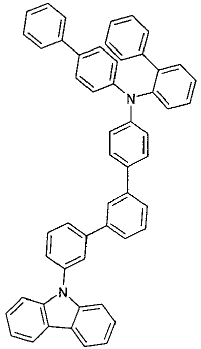 | 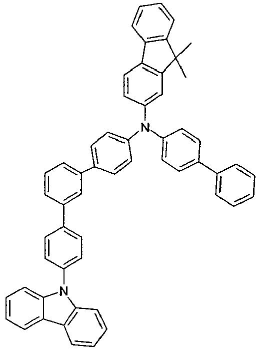 |
| HTM1 | HTM2 | HTM3 |
| 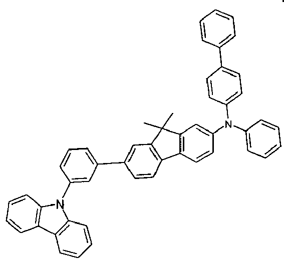 | 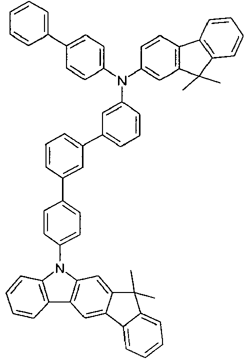 | |
| HTM4 | HTM5 | |

| Tabelle 2: Aufbau der elektronischen Vorrichtungen | | | | | | | |
|---|---|---|---|---|---|---|---|
| **Bsp.** | **HTL1** | **HTL2** | **HTL3** | **HTL4** | **EML** | **ETL** | **EIL** |
| | Dicke / nm | Dicke / nm | Dicke / nm | Dicke / nm | Dicke / nm | Dicke / nm | Dicke / nm |
| V1 | HIM1:F4TCNQ(3%) 20 nm | HIM1 155 nm | NPB:F4TCNQ(3%) 20 nm | NPB 20 nm | H1:SEB1(5%) 20 nm | ETM(50%):LiQ(50%) 30 nm | LiQ 1 nm |
| V2 | HIM1:F4TCNQ(3%) 20 nm | HIM1 155 nm | HTMV1:F4TCNQ(3%) 20 nm | HTMV1 20 nm | H1:SEB1(5%) 20 nm | ETM(50%):LiQ(50%) 30 nm | LiQ 1 nm |
| V3 | HIM1:F4TCNQ(3%) 20 nm | HIM1 155 nm | HTMV2:F4TCNQ(3%) 20 nm | HTMV2 20 nm | H1:SEB1(5%) 20 nm | ETM(50%):LiQ(50%) 30 nm | LiQ 1 nm |
| E1 | HIM1:F4TCNQ(3%) 20 nm | HIM1 155 nm | HTM1:F4TCNQ(3%) 20 nm | HTM1 20 nm | H1:SEB1(5%) 20 nm | ETM(50%):LiQ(50%) 30 nm | LiQ 1 nm |
| E2 | HIM1:F4TCNQ(3%) 20 nm | HIM1 155 nm | HTM2:F4TCNQ(3%) 20 nm | HTM2 20 nm | H1:SEB1(5%) 20 nm | ETM(50%):LiQ(50%) 30 nm | LiQ 1 nm |
| E3 | HIM1:F4TCNQ(3%) 20 nm | HIM1 155 nm | HTM3:F4TCNQ(3%) 20 nm | HTM3 20 nm | H1:SEB1(5%) 20 nm | ETM(50%):LiQ(50%) 30 nm | LiQ 1 nm |
| E4 | HIM1:F4TCNQ(3%) 20 nm | HIM1 155 nm | HTM4:F4TCNQ(3%) 20 nm | HTM4 20 nm | H1:SEB1(5%) 20 nm | ETM(50%):LiQ(50%) 30 nm | LiQ 1 nm |
| E5 | HIM1:F4TCNQ(3%) 20 nm | HIM1 155 nm | HTM5:F4TCNQ(3%) 20 nm | HTM5 20 nm | H1:SEB1(5%) 20 nm | ETM(50%):LiQ(50%) 30 nm | LiQ 1 nm |
| V4 | HIM1:F4TCNQ(3%) 20 nm | HIM1 210 nm | NPB:F4TCNQ(3%) 20 nm | NPB 20 nm | H2:TEG(10%) 30 nm | ETM(50%):LiQ(50%) 40 nm | LiQ 1 nm |
| V5 | HIM1:F4TCNQ(3%) 20 nm | HIM1 210 nm | HTMV1:F4TCNQ(3%) 20 nm | HTMV1 20 nm | H2:TEG(10%) 30 nm | ETM(50%):LiQ(50%) 40 nm | LiQ 1 nm |
| V6 | HIM1:F4TCNQ(3%) 20 nm | HIM1 210 nm | HTMV2:F4TCNQ(3%) 20 nm | HTMV2 20 nm | H2:TEG(10%) 30 nm | ETM(50%):LiQ(50%) 40 nm | LiQ 1 nm |
| E6 | HIM1:F4TCNQ(3%) 20 nm | HIM1 210 nm | HTM1:F4TCNQ(3%) 20 nm | HTM1 20 nm | H2:TEG(10%) 30 nm | ETM(50%):LiQ(50%) 40 nm | LiQ 1 nm |
| E7 | HIM1:F4TCNQ(3%) 20 nm | HIM1 210 nm | HTM2:F4TCNQ(3%) 20 nm | HTM2 20 nm | H2:TEG(10%) 30 nm | ETM(50%):LiQ(50%) 40 nm | LiQ 1 nm |
| E8 | HIM1:F4TCNQ(3%) 20 nm | HIM1 210 nm | HTM3:F4TCNQ(3%) 20 nm | HTM3 20 nm | H2:TEG(10%) 30 nm | ETM(50%):LiQ(50%) 40 nm | LiQ 1 nm |
| E9 | HIM1:F4TCNQ(3%) 20 nm | HIM1 210 nm | HTM5:F4TCNQ(3%) 20 nm | HTM5 20 nm | H2:TEG(10%) 30 nm | ETM(50%):LiQ(50%) 40 nm | LiQ 1 nm |

Es werden Vorrichtungen mit den erfindungsgemäßen Verbindungen HTM1 bis HTM5 hergestellt (E1 - E9). Weiterhin werden als Referenz Vorrichtungen mit den im Stand der Technik bekannten Verbindungen NPB, HTMV1 und HTMV2 hergestellt (V1 - V6).

Die Verbindungen werden als Lochtransportmaterialien bzw. als Elektronenblockiermaterialien in einer entsprechenden Schicht verwendet. Im Falle der Verwendung als Lochtransportmaterial sind die Verbindungen in den vorliegenden Beispielen mit einem p-Dotanden dotiert.

Die Verbindungen können jedoch auch in anderen Funktionen verwendet werden, beispielsweise als Lochtransportmaterialien ohne p-Dotierung oder als Hostmaterialien für phosphoreszierende Emitter.

### Beispiele V1-V3 und E1-E5

In einer fluoreszierenden OLED zeigen im Vergleich zu den Referenzproben V1 (6,2 %), V2 (7.2 %) und V3 (6,9 %) die erfindungsgemäßen Proben E1 mit 7,7 %, E2 mit 7,9 %, E3 mit 8,2%, E4 mit 8,5 % und E5 mit 7,6 % eine höhere Quanteneffizienz bei 10 mA/cm².

Auch ist die Lebensdauer LT80 bei 50 mA/cm² bei allen erfindungsgemäßen Proben E1 (260 h), E2 (285 h), E3 (290 h), E4 (290 h) und E5 (300 h) deutlich besser als bei den Referenzproben V1 (135 h), V2 (210 h) und V3 (160 h).

### Beispiele V4-V6 und E6-E9

In einer phosphoreszierenden OLED (grüne Emission) zeigen die Referenzproben V4 (11,7 %), V5 (17,6 %) und V6 (17,4 %) etwas niedrigere oder gleiche Quanteneffizienzen bei 2mA/cm² verglichen mit den erfindungsgemäßen Proben E6 (17,7 %), E7 (18,2 %), E8 (20,6 %) und E9 (19,1 %).

Auch sind die Lebensdauern bei 20 mA/cm² der erfindungsgemäßen Proben E6 (185 h), E7 (230 h), E8 (230 h), E9 (210 h) deutlich länger als bei den Referenzproben V4 (80 h), V5 (125 h) und V6 (115 h).

Zusammenfassend zeigen die erfindungsgemäßen Beispiele sehr gute Werte für die Quanteneffizienz und für die Lebensdauer, sowohl bei fluoreszierenden OLEDs als auch bei phosphoreszierenden OLEDs. Weiterhin zeigen die erfindungsgemäßen Beispiele Vorteile in den genannten Punkten gegenüber OLEDs, die im Stand der Technik bekannte Verbindungen als funktionelle Materialien enthalten.

## Patentansprüche

1. Verbindung einer Formel (I) 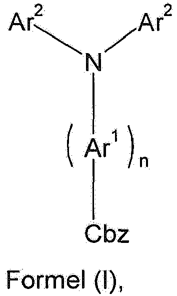 wobei gilt:
Cbz ist eine Carbazolgruppe, die mit einem oder mehreren Resten R¹ substituiert sein kann, und die über das Carbazol-Stickstoffatom gebunden ist;
Ar¹ ist bei jedem Auftreten gleich oder verschieden eine Arylgruppe mit 6 aromatischen Ringatomen, die mit einem oder mehreren Resten R² substituiert sein kann, wobei einzelne Gruppen Ar¹ miteinander über Reste R² verbunden sein können;
Ar² ist bei jedem Auftreten gleich oder verschieden ein aromatisches oder heteroaromatisches Ringsystem mit 6 bis 30 aromatischen Ringatomen, das mit einem oder mehreren Resten R² substituiert sein kann;
R¹, R² ist bei jedem Auftreten gleich oder verschieden H, D, F, Cl, Br, I, C(=O)R³, CN, Si(R³)₃, P(=O)(R³)₂, S(=O)R³, S(=O)₂R³, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch - R³C=CR³-, -C≡C-, Si(R³)₂, C=O, C=S, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I oder CN ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann, wobei zwei oder mehr Reste R¹ bzw. R² miteinander verknüpft sein können und einen Ring bilden können;
R³ ist bei jedem Auftreten gleich oder verschieden H, D, F, Cl, Br, I, C(=O)R⁴, CN, Si(R⁴)₃, N(R⁴)₂, P(=O)(R⁴)₂, S(=O)R⁴, S(=O)₂R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch-R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können und wobei ein oder mehrere H-Atome in den oben genannten Gruppen durch D, F, Cl, Br, I oder CN ersetzt sein können, oder ein aromatisches oder heteroaromatisches Ringsystem mit 5 bis 30 aromatischen Ringatomen, das jeweils durch einen oder mehrere Reste R⁴ substituiert sein kann, oder eine Aryloxy- oder Heteroaryloxygruppe mit 5 bis 30 aromatischen Ringatomen, die durch einen oder mehrere Reste R⁴ substituiert sein kann, wobei zwei oder mehr Reste R³ miteinander verknüpft sein können und einen Ring bilden können;
R⁴ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R⁴ miteinander verknüpft sein und einen Ring bilden;
n ist 3;
wobei die Verbindung der Formel (I) neben der Gruppe Cbz keine weitere Carbazolgruppe enthält,
und wobei mindestens eine Gruppe Ar¹ in der Verbindung der Formel (I) gewählt ist aus ortho-Phenylen oder meta-Phenylen, wobei die Gruppen mit einem oder mehreren Resten R² substituiert sein können.

2. Verbindung nach Anspruch 1, **dadurch gekennzeichnet, dass** die Verbindung der Formel (I) neben der gezeigten Arylaminogruppe keine weitere Arylaminogruppe enthält.

3. Verbindung nach Anspruch 1 oder 2, **dadurch gekennzeichnet, dass** die Gruppe Cbz gewählt ist aus Gruppen der Formel (Cbz) 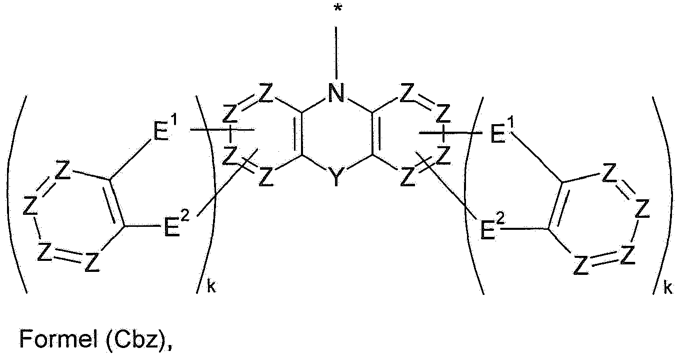 wobei gilt:
Z ist gleich CR¹, wobei Z gleich C ist, wenn eine Gruppe E¹ oder E² gebunden ist;
E¹, E² ist bei jedem Auftreten gleich oder verschieden gewählt aus einer Einfachbindung, C(R¹)₂, Si(R¹)₂, C=O, O, S, S=O, SO₂ und NR¹, wobei E¹ und E² nicht beide eine Einfachbindung sein können;
Y ist eine Einfachbindung;
k ist bei jedem Auftreten gleich oder verschieden 0 oder 1; und
wobei die Gruppe der Formel (Cbz) über die mit * markierte Bindung gebunden ist.

4. Verbindung nach Anspruch 3, **dadurch gekennzeichnet, dass** in der Gruppe der Formel (Cbz) innerhalb einer Einheit in Klammern mit Index k eine von E¹ und E² eine Einfachbindung ist, und die andere gewählt ist aus C(R¹)₂, Si(R¹)₂, C=O, O, S, S=O, SO₂ und NR¹.

5. Verfahren zur Herstellung einer Verbindung gemäß Formel (I) nach einem oder mehreren der Ansprüche 1 bis 4, **dadurch gekennzeichnet, dass** ein Carbazolderivat in einer Kupplungsreaktion mit einer Arylverbindung umgesetzt wird.

6. Formulierung, enthaltend mindestens eine Verbindung gemäß Formel (I) nach einem oder mehreren der Ansprüche 1 bis 4 sowie mindestens ein Lösungsmittel.

7. Verwendung einer Verbindung gemäß Formel (I) nach einem oder mehreren der Ansprüche 1 bis 4 in einer elektronischen Vorrichtung.

8. Elektronische Vorrichtung, enthaltend mindestens eine Verbindung gemäß Formel (I) nach einem oder mehreren der Ansprüche 1 bis 4.

9. Elektronische Vorrichtung nach Anspruch 8, **dadurch gekennzeichnet, dass** sie gewählt ist aus aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs).

10. Organische Elektrolumineszenzvorrichtung nach Anspruch 9, **dadurch gekennzeichnet, dass** die Verbindung gemäß Formel (I) in einer Lochtransportschicht, einer Elektronenblockierschicht, einer Lochinjektionsschicht oder in einer emittierenden Schicht enthalten ist.

## Claims

1. Compound of a formula (I) 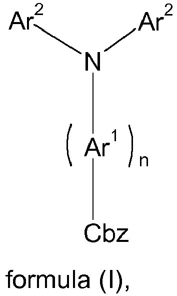 where:
Cbz is a carbazole group, which may be substituted by one or more radicals R¹, and which is bonded via the carbazole nitrogen atom;
Ar¹ is on each occurrence, identically or differently, an aryl group having 6 aromatic ring atoms, which may be substituted by one or more radicals R², where individual groups Ar¹ may be connected to one another via radicals R²;
Ar² is on each occurrence, identically or differently, an aromatic or heteroaromatic ring system having 6 to 30 aromatic ring atoms, which may be substituted by one or more radicals R²;
R¹, R² are on each occurrence, identically or differently, H, D, F, Cl, Br, I, C(=O)R³, CN, Si(R³)₃, P(=O)(R³)₂, S(=O)R³, S(=O)₂R³, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R³ and where one or more CH₂ groups in the above-mentioned groups may be replaced by -R³C=CR³-, -C≡C-, Si(R³)₂, C=O, C=S, C=NR³, -C(=O)O-, -C(=O)NR³-, NR³, P(=O)(R³), -O-, -S-, SO or SO₂ and where one or more H atoms in the above-mentioned groups may be replaced by D, F, Cl, Br, I or CN, or an aromatic or heteroaromatic ring system having 5 to 30 aromatic ring atoms, which may in each case be substituted by one or more radicals R³, or an aryloxy or heteroaryloxy group having 5 to 30 aromatic ring atoms, which may be substituted by one or more radicals R³, where two or more radicals R¹ or R² may be linked to one another and may form a ring;
R³ is on each occurrence, identically or differently, H, D, F, Cl, Br, I, C(=O)R⁴, CN, Si(R⁴)₃, N(R⁴)₂, P(=O)(R⁴)₂, S(=O)R⁴, S(=O)₂R⁴, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R⁴ and where one or more CH₂ groups in the above-mentioned groups may be replaced by -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO or SO₂ and where one or more H atoms in the above-mentioned groups may be replaced by D, F, Cl, Br, I or CN, or an aromatic or heteroaromatic ring system having 5 to 30 aromatic ring atoms, which may in each case be substituted by one or more radicals R⁴, or an aryloxy or heteroaryloxy group having 5 to 30 aromatic ring atoms, which may be substituted by one or more radicals R⁴, where two or more radicals R³ may be linked to one another and may form a ring;
R⁴ is on each occurrence, identically or differently, H, D, F or an aliphatic, aromatic or heteroaromatic organic radical having 1 to 20 C atoms, in which, in addition, one or more H atoms may be replaced by D or F; two or more substituents R⁴ here may be linked to one another and may form a ring;
n is 3;
where the compound of the formula (I) contains no further carbazole group besides the group Cbz,
and where at least one group Ar¹ in the compound of the formula (I) is selected from ortho-phenylene or meta-phenylene, where the groups may be substituted by one or more radicals R².

2. Compound according to Claim 1, **characterised in that** the compound of the formula (I) contains no further arylamino group besides the arylamino group shown.

3. Compound according to Claim 1 or 2, **characterised in that** the group Cbz is selected from groups of the formula (Cbz) 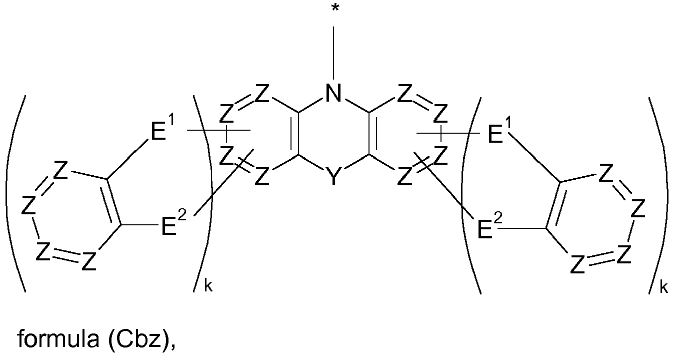 where:
Z is equal to CR¹, where Z is equal to C if a group E¹ or E² is bonded;
E¹, E² are selected on each occurrence, identically or differently, from a single bond, C(R¹)₂, Si(R¹)₂, C=O, O, S, S=O, SO₂ and NR¹, where E¹ and E² cannot both be a single bond;
Y is a single bond,
k is on each occurrence, identically or differently, 0 or 1; and
where the group of the formula (Cbz) is bonded via the bond labelled with *.

4. Compound according to Claim 3, **characterised in that** in the group of the formula (Cbz) within a unit in brackets with index k, one of E¹ and E² is a single bond and the other is selected from C(R¹)₂, Si(R¹)₂, C=O, O, S, S=O, SO₂ and NR¹.

5. Process for the preparation of a compound of the formula (I) according to one or more of Claims 1 to 4, **characterised in that** a carbazole derivative is reacted with an aryl compound in a coupling reaction.

6. Formulation comprising at least one compound of the formula (I) according to one or more of Claims 1 to 4 and at least one solvent.

7. Use of a compound of the formula (I) according to one or more of Claims 1 to 4 in an electronic device.

8. Electronic device containing at least one compound of the formula (I) according to one or more of Claims 1 to 4.

9. Electronic device according to Claim 8, **characterised in that** it is selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs).

10. Organic electroluminescent device according to Claim 9, **characterised in that** the compound of the formula (I) is present in a hole-transport layer, an electron-blocking layer, a hole-injection layer or in an emitting layer.

## Revendications

1. Composé d'une formule (I) : 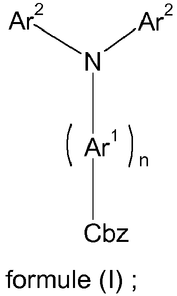 dans laquelle :
Cbz est un groupe carbazole, lequel peut être substitué par un radical ou par plusieurs radicaux R¹, et lequel est lié via l'atome d'azote ;
Ar¹ est pour chaque occurrence, de manière identique ou différente, un groupe aryle qui comporte 6 atomes de cycle aromatique, lequel peut être substitué par un radical ou par plusieurs radicaux R², où des groupes individuels Ar¹ peuvent être connectés les uns aux autres via des radicaux R²;
Ar² est pour chaque occurrence, de manière identique ou différente, un système de cycle aromatique ou hétéroaromatique qui comporte de 6 à 30 atomes de cycle aromatique, lequel peut être substitué par un radical ou par plusieurs radicaux R² ;
R¹, R² sont pour chaque occurrence, de manière identique ou différente, H, D, F, Cl, Br, I, C(=O)R³, CN, Si(R³)₃, P(=O)(R³)₂, S(=O)R³, S(=O)₂R³, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R³ et où un ou plusieurs groupe(s) CH₂ dans les groupes qui ont été mentionnés ci-avant peut/peuvent être remplacé(s) par -R³C=CR³-, -C≡C-, Si(R³)₂, C=O, C=S, C=NR³, -C(=O)O-, -C(=O)NR³-, NR³, P(=O)(R³), -O-, -S-, SO ou SO₂ et où un ou plusieurs atome(s) de H dans les groupes qui ont été mentionnés ci-avant peut/peuvent être remplacé(s) par D, F, Cl, Br, I ou CN, ou un système de cycle aromatique ou hétéroaromatique qui comporte de 5 à 30 atomes de cycle aromatique, lequel peut dans chaque cas être substitué par un radical ou par plusieurs radicaux R³, ou un groupe aryloxy ou hétéroaryloxy qui comporte de 5 à 30 atomes de cycle aromatique, lequel peut être substitué par un radical ou par plusieurs radicaux R³, où deux radicaux R¹ ou R² ou plus peuvent être liés l'un à l'autre ou les uns aux autres et peuvent former un cycle ;
R³ est pour chaque occurrence, de manière identique ou différente, H, D, F, Cl, Br, I, C(=O)R⁴, CN, Si(R⁴)₃, N(R⁴)₂, P(=O)(R⁴)₂, S(=O)R⁴, S(=O)₂R⁴, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R⁴ et où un ou plusieurs groupe(s) CH₂ dans les groupes qui ont été mentionnés ci-avant peut/peuvent être remplacé(s) par -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO ou SO₂ et où un ou plusieurs atome(s) de H dans les groupes qui ont été mentionnés ci-avant peut/peuvent être remplacé(s) par D, F, Cl, Br, I ou CN, ou un système de cycle aromatique ou hétéroaromatique qui comporte de 5 à 30 atomes de cycle aromatique, lequel peut dans chaque cas être substitué par un radical ou par plusieurs radicaux R⁴, ou un groupe aryloxy ou hétéroaryloxy qui comporte de 5 à 30 atomes de cycle aromatique, lequel peut être substitué par un radical ou par plusieurs radicaux R⁴, où deux radicaux R³ ou plus peuvent être liés l'un à l'autre ou les uns aux autres et peuvent former un cycle ;
R⁴ est pour chaque occurrence, de manière identique ou différente, H, D, F ou un radical organique aliphatique, aromatique ou hétéroaromatique qui comporte de 1 à 20 atome(s) de C, où, en outre, un ou plusieurs atome(s) de H peut/ peuvent être remplacé(s) par D ou F ; deux substituants R⁴ ou plus peuvent ici être liés l'un à l'autre ou les uns aux autres et peuvent former un cycle ;
n est 3 ;
où le composé de la formule (I) ne contient pas d'autre groupe carbazole en plus du groupe Cbz ;
et où au moins un groupe Ar¹ dans le composé de la formule (I) est sélectionné parmi ortho-phénylène ou méta-phénylène, où les groupes peuvent être substitués par un radical ou par plusieurs radicaux R².

2. Composé selon la revendication 1, **caractérisé en ce que** le composé de la formule (I) ne contient pas d'autre groupe arylamino en plus du groupe arylamino présenté.

3. Composé selon la revendication 1 ou 2, **caractérisé en ce que** le groupe Cbz est sélectionné parmi les groupes de la formule (Cbz) : 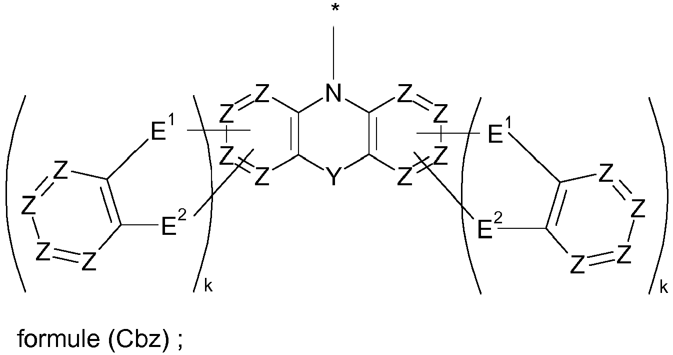 dans laquelle :
Z est égal à CR¹, où Z est égal à C si un groupe E¹ ou E² est lié ;
E¹, E² sont sélectionnés pour chaque occurrence, de manière identique ou différente, parmi une liaison simple, C(R¹)₂, Si(R¹)₂, C=O, O, S, S=O, SO₂ et NR¹, où E¹ et E² ne peuvent pas être tous les deux une liaison simple ;
Y est une liaison simple ;
k est pour chaque occurrence, de manière identique ou différente, 0 ou 1 ; et
où le groupe de la formule (Cbz) est lié via la liaison qui est étiquetée à l'aide de *.

4. Composé selon la revendication 3, **caractérisé en ce que**, dans le groupe de la formule (Cbz) à l'intérieur d'une unité entre parenthèses avec l'indice k, l'un de E¹ et E² est une liaison simple et l'autre est sélectionné parmi C(R¹)₂, Si(R¹)₂, C=O, O, S, S=O, SO₂ et NR¹.

5. Procédé pour la préparation d'un composé de la formule (I) selon une ou plusieurs des revendications 1 à 4, **caractérisé en ce qu'**un dérivé de carbazole est amené à réagir avec un composé aryle selon une réaction de couplage.

6. Formulation comprenant au moins un composé de la formule (I) selon une ou plusieurs des revendications 1 à 4 et au moins un solvant.

7. Utilisation d'un composé de la formule (I) selon une ou plusieurs des revendications 1 à 4 dans un dispositif électronique.

8. Dispositif électronique contenant au moins un composé de la formule (I) selon une ou plusieurs des revendications 1 à 4.

9. Dispositif électronique selon la revendication 8, **caractérisé en ce qu'**il est sélectionné parmi le groupe qui est constitué par les circuits intégrés organiques (OIC), les transistors à effet de champ organiques (OFET), les transistors à film mince organiques (OTFT), les transistors à émission de lumière organiques (OLET), les cellules solaires organiques (OSC), les détecteurs optiques organiques, les photorécepteurs organiques, les dispositifs à extinction de champ organiques (OFQD), les cellules électrochimiques à émission de lumière organiques (OLEC), les diodes laser organiques (O-laser) et les dispositifs électroluminescents organiques (OLED).

10. Dispositif électroluminescent organique selon la revendication 9, **caractérisé en ce que** le composé de la formule (I) est présent dans une couche de transport de trous, une couche de blocage d'électrons, une couche d'injection de trous ou dans une couche d'émission.
